# EUROPEAN PATENT APPLICATION

(11) **EP 4 697 197 A1**
(43) Date of publication of application: **18.02.2026**
(21) Application number: 24194173.1
(22) Date of filing: 12.08.2024
(51) Int. Cl.: G06F 16/3329, G06F 16/353, G16H 50/20, G06F 16/901

(54) **CONTEXT-BASED CLINICAL KNOWLEDGE EXTRACTION AND DOCUMENT TRANSMISSION**

(71) Applicant: Dyad AI, Inc., London EC2A 4NE (GB)
(72) Inventor: HAMBLIN, Steven, London (GB); PARVIZI, Artemis, London (GB); TAYLER, Alexander, London (GB)
(74) Representative: Strange, Harry George

(57) **Abstract**

A method for context-based clinical knowledge extraction and automatic transmission of clinical documents. A text-based representation of a document having a clinical context is obtained and an identifier which uniquely identifies the clinical context of the document is determined. An executable coding graph is identified from a plurality of executable coding graphs based on the identifier of the clinical context. The executable coding graph is indicative of a procedure for coding the document according to the clinical context and comprises a network of branch nodes interconnected with a plurality of coding nodes thereby forming a directed acyclic graph. The executable coding graph is executed on the text based representation of the document thereby generating a structured set of clinical information linked to the document enabling the automatic transmission of a clinical document based on the data extracted from the clinical document.

## Description

### TECHNICAL FIELD

The present disclosure relates to semantic data extraction and dataset generation. Particularly, but not exclusively, the present disclosure relates to context-driven semantic data extraction from a clinical document; and more particularly, but not exclusively, to the automatic transmission of a clinical document based on the data extracted from the clinical document.

### BACKGROUND

Interoperability of clinical, or medical, data is a key barrier to the efficient implementation of healthcare systems. Modern healthcare settings involve practitioners and healthcare providers which utilise different systems, protocols, and means for communicating clinical data. This difference in technology makes integrating data across providers difficult. Moreover, a lack of standards within clinical documents, as well as issues arising from a lack of resource to process and manage such documents, make processing, routing, and actioning clinical documents and clinical data inefficient and ineffective.

There is therefore a need for improved systems to automate processing of clinical documents which allows integration of data between healthcare systems.

### SUMMARY OF DISCLOSURE

According to an aspect of the present disclosure there is provided a method for context-based clinical knowledge extraction. The method comprises obtaining, by one or more processors, a text-based representation of a document having a clinical context, determining, by the one or more processors, an identifier which uniquely identifies the clinical context of the document by providing one or more portions of the text-based representation of the document to a classifier trained to generate a predicted identifier from text provided as input, identifying, by the one or more processors, an executable coding graph from a plurality of executable coding graphs based on the identifier of the clinical context, the executable coding graph indicative of a procedure for coding the document according to the clinical context and comprising a network of branch nodes interconnected with a plurality of coding nodes thereby forming a directed acyclic graph, wherein: a branch node of the network of branch nodes is operable to determine which node connected to the branch node is to be executed next according to an evaluation of a query related to the clinical context and linked to the branch node, wherein the query is evaluated based on a semantic analysis of the text based representation of the document, and a coding node of the plurality of coding nodes is operable to assign a clinical datum to a structured set of clinical information linked to the document, wherein the clinical datum is determined from the semantic analysis performed by executing a prior sequence of branch nodes connected to the coding node, and executing, by the one or more processors, the executable coding graph on the text based representation of the document thereby generating the structured set of clinical information linked to the document.

According to an additional aspect of the present disclosure there is provided a computer-readable medium storing instructions which, when executed by one or more processors, cause the one or more processors to carry out the method of obtaining a text-based representation of a document having a clinical context, determining an identifier which uniquely identifies the clinical context of the document by providing one or more portions of the text-based representation of the document to a classifier trained to generate a predicted identifier from text provided as input, identifying an executable coding graph from a plurality of executable coding graphs based on the identifier of the clinical context, the executable coding graph indicative of a procedure for coding the document according to the clinical context and comprising a network of branch nodes interconnected with a plurality of coding nodes thereby forming a directed acyclic graph, wherein: a branch node of the network of branch nodes is operable to determine which node connected to the branch node is to be executed next according to an evaluation of a query related to the clinical context and linked to the branch node, wherein the query is evaluated based on a semantic analysis of the text-based representation of the document, and a coding node of the plurality of coding nodes is operable to assign a clinical datum to a structured set of clinical information linked to the document, wherein the clinical datum is determined from the semantic analysis performed by executing a prior sequence of branch nodes connected to the coding node, and executing the executable coding graph on the text based representation of the document thereby generating the structured set of clinical information linked to the document.

According to a further aspect of the present disclosure there is provided a system comprising a memory storing a text-based representation of a document having a clinical context, a document classification module comprising a classifier trained to output a predicted clinical identifier from text provided as input, a coding graph module configured to identify a respective executable coding graph from a plurality of executable coding graphs based on a respective clinical context, wherein each of the plurality of executable coding graphs is indicative of a procedure for coding a clinical document according to a corresponding clinical context and comprises a network of branch nodes interconnected with a plurality of coding nodes thereby forming a directed acyclic graph, wherein a branch node of the network of branch nodes is operable to determine which node connected to the branch node is to be executed next according to an evaluation of a query related to the corresponding clinical context and linked to the branch node, wherein the query is evaluated based on a semantic analysis of the clinical document, and a coding node of the plurality of coding nodes is operable to assign a clinical datum to a structured set of clinical information linked to the clinical document, wherein the clinical datum is determined from the semantic analysis performed by executing a prior sequence of branch nodes connected to the coding node, and an orchestration module configured to provide one or more portions of the text-based representation of the document to the document classification module to determine an identifier for the document, provide the identifier to the coding graph module to identify an executable coding graph for the clinical context, and execute the executable coding graph on the text based representation of the document thereby generating the structured set of clinical information linked to the document.

Further aspects and embodiments of the present disclosure are set out in the appended claims. Advantages will become more apparent to those of ordinary skill in the art from the following description of the preferred embodiments which have been shown and described by way of illustration. As will be realized, the present embodiments may be capable of other and different embodiments, and their details are capable of modification in various respects. Accordingly, the drawings and description are to be regarded as illustrative in nature and not as restrictive.

### BRIEF DESCRIPTION OF DRAWINGS

Embodiments of the present disclosure will now be described, by way of example only, and with reference to the accompanying drawings, in which:
Figures 1A-1E show methods for clinical knowledge extraction according to embodiments of the present disclosure;
Figure 2 shows a document having a clinical context according to an embodiment of the present disclosure;
Figure 3 shows a structured set of clinical information extracted from the document shown in Figure 2 according to an embodiment of the present disclosure;
Figure 4A shows a portion of an executable coding graph according to an embodiment of the present disclosure;
Figure 4B shows an executable coding graph according to an embodiment of the present disclosure;
Figure 5 shows a clinical class model according to an embodiment of the present disclosure;
Figures 6A-6C show graphs generated from a structured set of clinical information according to an embodiment of the present disclosure;
Figure 7A-7B illustrate example user interfaces according to embodiments of the present disclosure;
Figure 8 shows a system according to an aspect of the present disclosure; and
Figure 9 shows an example computing system according to embodiments of the present disclosure.

### DETAILED DESCRIPTION

The ability to extract clinical information accurately and efficiently from a clinical document is important for numerous downstream tasks such as medical knowledge discovery, system automation, document obfuscation, automated document generation, and the like. Additionally, transforming clinical information within an unstructured document into a structured form allows for data to be efficiently and seamlessly integrated across different medical systems. The present disclosure is directed to context-driven clinical knowledge extraction for automated generation of structured clinical data from unstructured sources.

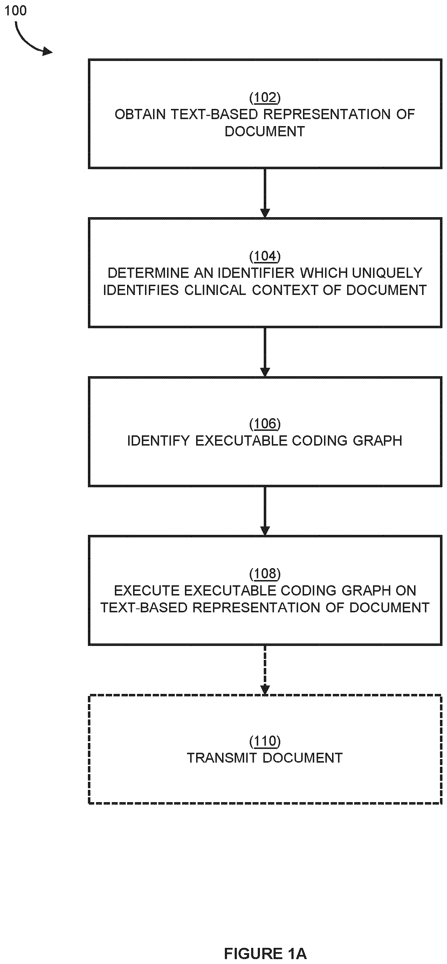

Figure 1A shows a method 100 for context-based clinical knowledge extraction according to an aspect of the present disclosure.

The method 100 comprises the steps of obtaining 102 a text-based representation of a document, determining 104 an identifier which uniquely identifies the clinical context of the document, identifying 106 an executable coding graph, and executing 108 the executable coding graph on the text-based representation of the document. The method 100 also comprises the optional step of transmitting 110 the document.

At the step of obtaining 102, a text-based representation of a document having a clinical context is obtained.

Here, a document having a clinical context can be a letter, email, communication, or the like which is related to a clinical setting or context. For example, a referral letter from a healthcare practitioner such as a doctor or a discharge summary from a hospital. The document is unstructured-that is, the document data is not stored, represented, or organised according to a data model or in a defined manner. As will be described in more detail below, a clinical context refers to the context (e.g., origin, purpose, etc.) of the document specifically within the clinical or healthcare setting. The clinical context of a document is linked to a clinical domain of the document-e.g., the healthcare department to which the document relates, such as A&E, haematology, or the like-and a type of the document-e.g., a discharge letter, a letter reporting test results, etc.

The document can be an electronic file comprising editable text (i.e., non-image based text) which can be directly extracted to generate a text-based representation of the document. Examples of such documents include e-mails, text files, word processor files, and the like. Alternatively, the document can be a digital scan of a physical document (e.g., a letter). In such examples, the document is an image or non-text based representation which needs to be converted into a text-based representation to allow semantic content to be extracted. Examples of such documents include image files (e.g., JPG, TIFF, etc.) or PDF files. The text-based representation of the document is obtained by an optical character recognition (OCR) process or a multi-modal generative model. Here, a multi-modal generative model is a generative model, such as a large language model (LLM), which is operable to process and generate content across multiple modalities (e.g., text, images, etc.). An example of a multi-modal generative model is the GPT-4o model provided by OpenAI. Advantageously, converting non-text based representations of a document to a text-based representation using a multi-model generative model preserves structural elements within the document, such as tables, which can help improve the extraction of features from such structural elements (e.g., blood test results, diagnoses, etc.).

At the step of determining 104, an identifier which uniquely identifies the clinical context of the document is determined by providing one or more portions of the text-based representation of the document to a classifier trained to generate a predicted identifier from text provided as input.

The methods and systems of the present disclosure are configured to handle documents from a range of different clinical contexts; however, the way in which a document is processed is dependent upon the clinical context. Therefore, the identification of the clinical context of a document allows the correct processing (i.e., the processing specific to the identified clinical context) to be applied to the document. As stated above, the clinical context of a document is linked to a clinical domain of the document and a type of the document. The clinical domain of the document and the type of the document form a pair which uniquely identifies the clinical context. In one embodiment, each clinical domain and document type pairing is assigned a unique numerical identifier.

A classifier is trained to identify an identifier which uniquely identifies the clinical context of a document from one or more portions of a text-based representation of the document provided as input. The identifier can be the unique numerical identifier referred to above or the clinical domain and the type of the document. In one embodiment, two classifiers are used to identify the clinical domain and the type of the document separately.

The classifier is any suitable natural language processing or machine learning model. For example, the classifier can be a probabilistic model which assigns a probability score to each clinical context based on the presence of specific keywords and/or key phrases within the text-based representation of the document. The document can then be assigned to the clinical context having the highest probability score. As a further example, the classifier can be a machine learning model trained to predict an identifier of the clinical context from one or more portions of the text-based representation. Examples include multi-layer perceptrons, supervised topic modelling, and the like which are trained on a training data set of documents having known clinical contexts. In one embodiment, the classifier is a large language model (LLM) which is provided with a prompt operable to cause the LLM to infer a clinical context from the text-based representation provided as part of the prompt. For example, the prompt may comprise a command portion including an instruction to the LLM regarding the task to be performed, the text-based representation of the document, and a list of clinical contexts (clinical domain and document type pairs).

At the step of identifying 106, an executable coding graph is identified from a plurality of executable coding graphs based on the identifier of the clinical context. The executable coding graph is indicative of a procedure for coding the document according to the clinical context and comprises a network of branch nodes interconnected with a plurality of coding nodes thereby forming a directed acyclic graph.

In general, an executable coding graph encodes a set of operations for extracting structured clinical data from unstructured document data. An executable coding graph is context-specific in that the operations for extracting relevant semantic content from a document are localised to the clinical context of the document. This helps improve the accuracy of the knowledge extraction process which improves the robustness and fidelity of the data subsequently generated thereby improving the performance of downstream processes which rely on such data.

Each clinical context is associated with, or linked to, at least one executable coding graph such that the step of identifying 106 comprises obtaining the executable coding graph associated with the previously identified clinical context. In general, an executable coding graph is a structured set of processes, or operations, which are executed on the text-based representation of the document to extract semantic information from the text-based representation in the form of clinical data. Because each executable coding graph is specific to a clinical context, the operations in each executable coding graph are specific to the clinical context thereby enabling the clinical data to be efficiently and accurately extracted.

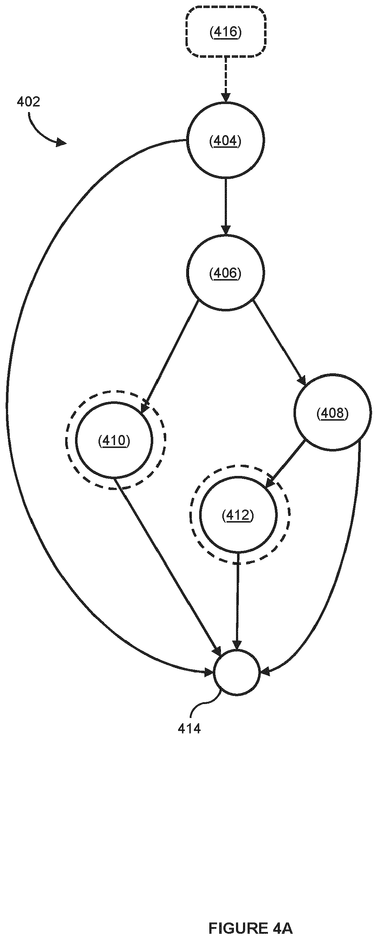

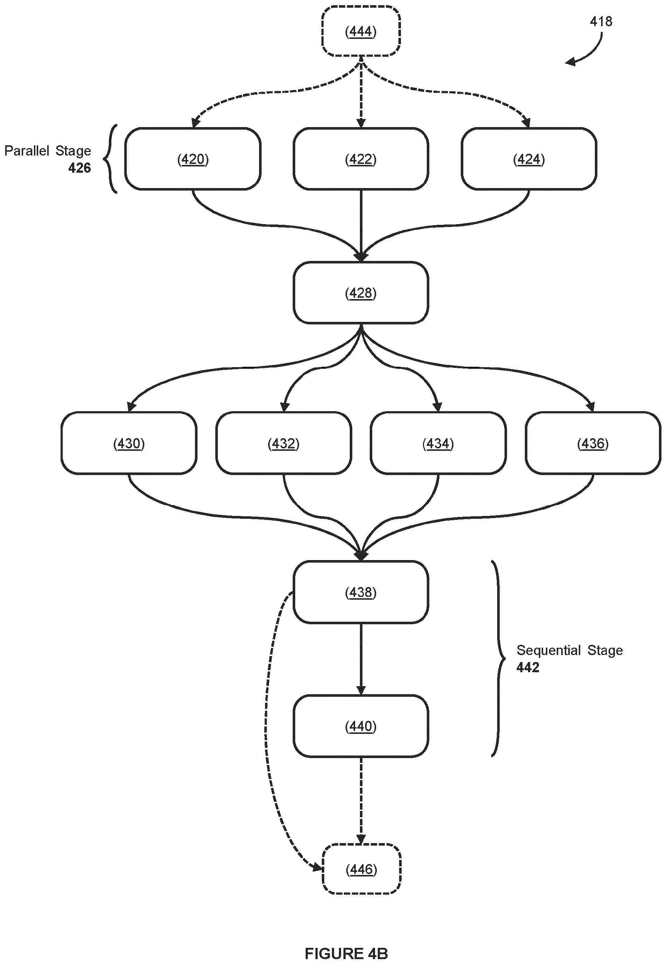

As will be described in more detail below in relation to Figures 4A and 4B, an executable coding graph is composed of branch nodes and coding nodes which are grouped into processes or process blocks. A branch node (alternatively referred to as an extraction node, a conditional node, a support node, or a non-coding node) is operable to determine which node connected to the branch node is to be executed next according to an evaluation of a query related to the clinical context and linked to the branch node. The query is evaluated based on a semantic analysis of the text based representation of the document. As such, a branch node determines from the semantic content of the text-based representation what information is present within the text-based representation and so helps determine what coding should be applied. A coding node (alternatively referred to as a code node, an execution node, an action node, or an operation node) is operable to assign a clinical datum to a structured set of clinical information linked to the document. The clinical datum is determined from the semantic analysis performed by executing a prior sequence of branch nodes connected to the coding node.

Therefore, a coding node can be supported by a preceding network of branch nodes which determine the coding to be applied based on a semantic analysis of the text-based representation of the document. The coding node and the preceding network of branch nodes can be grouped into a process linked to the coding.

The executable coding graph can be represented using a scripting language, as a graph-based representation format (e.g., RDF-based triples), or a markup language. For example, the executable coding graph can be a JSON file, a YAML file, a TTL file, an XML file or the like defining the structure of the executable coding graph and referencing processing logic (e.g., functions, external API calls, etc.) to perform the operations at each node.

At the step of executing 108, the executable coding graph is executed on the text based representation of the document thereby generating the structured set of clinical information linked to the document.

Executing the executable coding graph comprises parsing, interpreting, or executing the structured set of nodes defined by the executable coding graph. As many of the operations in the executable coding graph perform a semantic analysis of the document, the text-based representation of the document is provided to the requisite nodes/functions/operations of the executable coding graph to extract the relevant data.

The output of executing the executable coding is the structured set of clinical information linked to the document. The structured set of clinical information (alternatively referred to as clinical data, structured data, or a structured clinical data set) comprises the clinical data, such as clinical coding, patient data, healthcare provider data, etc., extracted from the text-based representation of the document. Because the data extraction process performed by the executable coding graph is semantically driven, the extracted clinical data can be structured according to the type, meaning, and context of the data.

In one embodiment, the structured set of clinical information comprises a plurality of data tables each related to a clinical category. One or more of the data tables can include data extracted from the document by the method 100. A first data table comprises patient specific information including patient name, medical identifier (e.g., National Health Service (NHS) number), date of birth, postcode, and the like. A second data table comprises document specific information including the clinical domain (e.g., the department which generated the document), the type of the document, the date of sending of the document, and the clinical date referenced in the document. A third data table comprises encounter information where each row includes a clinical code (e.g., a SNOMED code) for a clinical encounter involving the patient, the portion of the document mentioning the encounter, and a date associated with the encounter. The date associated with the encounter can be a date in the past, a present date, or a date in the future. A fourth data table comprises diagnosis information where each row includes a clinical code (e.g., a SNOMED code) for a diagnosis mentioned within the document, the portion of the document mentioning the diagnosis, and a date associated with the diagnosis. A fifth data table comprises procedure information where each row includes a clinical code for a procedure mentioned within the document, the portion of the document mentioning the procedure, and a date associated with the procedure. A sixth data table comprises medicament (medication) information where each row includes a medication name or a clinical code for a medicament or medicine mentioned within the document, the portion of the document mentioning the medicament, and a date associated with the medicament. Additional information included in the sixth data table can include status information such as whether the medication is new, changed, stopped, or is current. A seventh data table comprises measurement information where each row includes a clinical code for a measurement (e.g., a blood test result, a weight, a height, etc.) mentioned within the document, the measurement mentioned within the document and the unit associated with the measurement, the portion of the document mentioning the measurement, and a date associated with the measurement. Additionally, the structured set of clinical information can include further data tables such as a blood values table, a social information table, a family history table, and/or an encounter summary table.

At the optional step of transmitting 110, the document is transmitted along a communication channel to a recipient entity associated with a clinical class identified from the structured set of clinical information linked to the document.

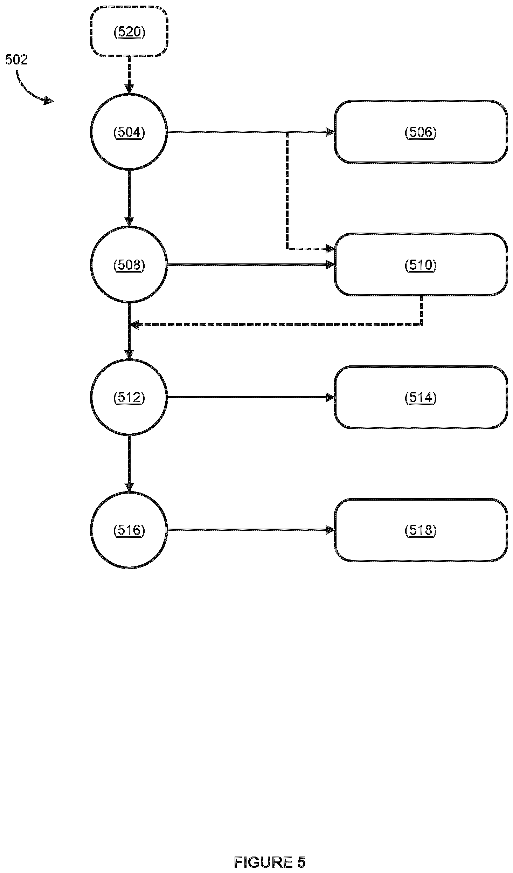

That is, based on the clinical data extracted from the document, the document is automatically routed to a recipient entity for further review, storage, processing, or the like (as illustrated in Figure 5 below). Advantageously, this allows a large volume of clinical documents (e.g., hundreds, thousands, tens of thousands, etc.) to be processed and routed automatically and without human intervention. This can lead to a significant increase in efficiency and a significant reduction in handle time in settings where thousands, if not tens or hundreds of thousands, of documents are inspected and routed daily. This in turn helps improve patient outcomes by ensuring that relevant data is accurately identified, logged, and acted upon quickly and efficiently.

Advantageously, this helps improve patient care and outcomes by enabling health care providers to extract and act upon clinical knowledge and information more quickly. This is particularly important in high risk clinical domains or where there are potential safeguarding concerns which need to be identified quickly in order to enable fast and appropriate patient care.

In one embodiment, the step of transmitting 110 is performed automatically after, or as part of, the execution of the executable coding graph. That is, the executable coding graph can comprise a final node which causes the document and/or the structured set of clinical data to be automatically transmitted along the relevant communication channel (e.g., email, secure communication channel, file transfer protocol, and the like).

The clinical class can be determined from the structured set of clinical information by a clinical class model. The clinical class model is a rule-based, statistical, or predictive (e.g., machine learning) learning model which is operable to process the structured set of clinical information to determine a clinical class (or clinical risk class) associated with the document.

In one embodiment, and as described in more detail in relation to Figure 5 below, the clinical class model comprises a sequence of clinical gates, where a clinical gate has a criterion and is linked to one of a plurality of clinical classes which is assigned to the document if the structured set of clinical information satisfies the criterion of the clinical gate. A clinical gate identifies one or more characteristics, properties, values, or elements of the structured set of clinical information which may indicate that the document belongs to the clinical class associated with the clinical gate. If no such indicating features are found within the structured set of clinical information, the process proceeds to the next clinical gate in the clinical class model. In this way, the clinical class model represents a cascaded sequence of clinical gates, or clinical risk gates, which are used to classify the clinical class of a document efficiently and quickly.

In an alternative embodiment, the clinical class model is a prediction model (e.g., a machine learning model or a statistical model) trained to generate a predicted clinical class from one or more portions of the structured set of clinical information.

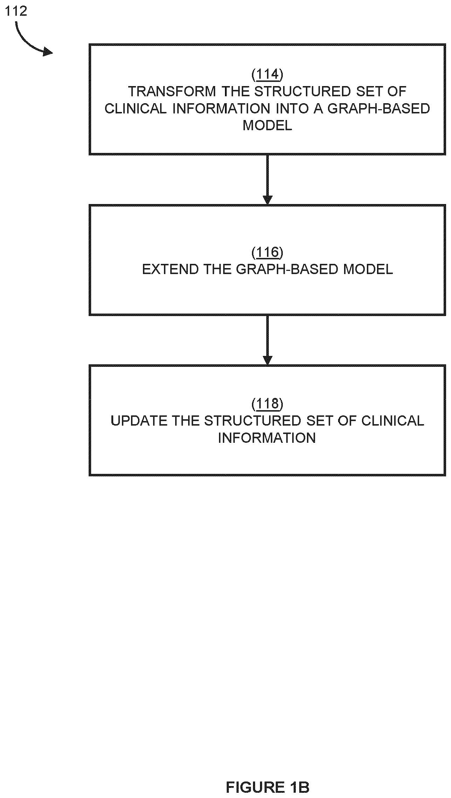

Figure 1B shows a sequence of steps 112 for updating a structured set of clinical information according to an embodiment of the present disclosure.

The sequence of steps 112 comprises transforming 114 the structured set of clinical information into a graph-based model, extending 116 the graph-based model, and updating 118 the structured set of clinical information. In one embodiment, the structured set of clinical information is generated by the method 100 shown in Figure 1A. As such, the sequence of steps 112 can be performed as part of the steps described in relation to Figure 1A above (e.g., by performing the sequence of steps 112 after executing 108 the executable coding graph to generate the structured set of clinical information).

At the step of transforming 114, the structured set of clinical information is transformed into a graph-based model.

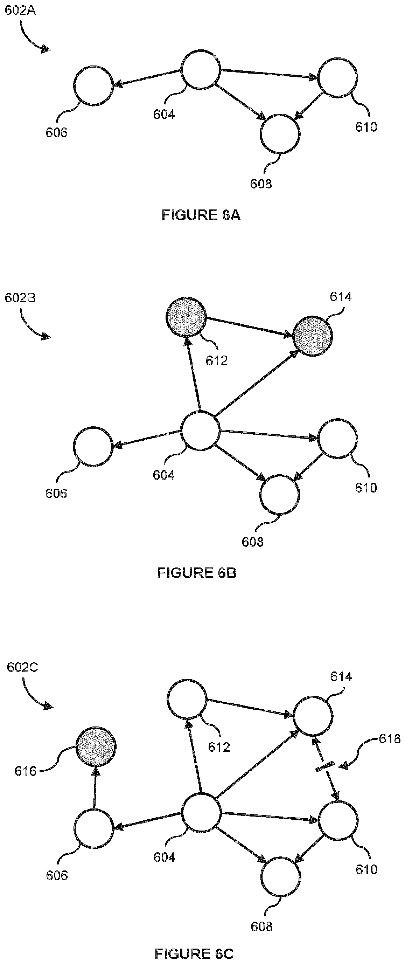

The graph-based model (alternatively referred to as a graph, a graph model, or a clinical graph) represents the clinical, biomedical, and social determinants of health information contained within the structured set of clinical information within a graphical form (as shown in Figure 6A). As is known, a graph-based model is a graph comprising nodes (or vertices) coupled by edges (or links). A node within a graph-based model represents an entity within the structured set of clinical information, and an edge defines a relationship between two nodes (i.e., a relationship between two entities). A node can comprise a type and one or more attributes with accompanying attribute values. For example, a node representing the drug Anakinra would have a type of "medication" and attributes such as the medicament name ("Anakinra") and the clinical code for Anakinra (e.g., SNOMED code 395279009). An edge can be directed or undirected and can be associated with, or comprise, a relationship between the nodes connected by the edge. For example, a first node representing a patient can be coupled to a second node representing a disease by an edge which defines the relationship in the direction from the first to the second node as "diagnosed_with".

Therefore, a graph-based model provides a graphical representation of the semantic knowledge extracted from a document. Representing this knowledge within a graph allows further processing, modification, and extension of the graph to be performed whilst also enriching the data extracted from the document.

A graph-based model can be generated from the structured set of clinical information using any suitable algorithm or technique. For example, entities within the structured set of clinical information are converted to nodes with the graph-based model. An entity in the structured set of clinical information corresponds to a single row, or clinical datum, such as a patient, a diagnosis, etc. Alternatively, each row in the structured set of clinical information can be transformed into two or more entities. A node is assigned a type according to the clinical category from which the row, or clinical datum, was extracted (e.g., "patient" for a node generated from the "Patient Information" clinical category, "procedure" for a node generated from the "Procedure" clinical category, etc.). The values of the row, or clinical datum, are added as attributes to the node. For example, attributes for the patient's name, date of birth, medical number, and post code can be added for a patient node from the corresponding data held in the structured set of clinical information. Edges are then added to the graph-based model according to rules determining relationships between clinical categories. For example, for any diagnoses that exist for a patient, an edge is added from the patient node to each diagnosis node with the edge attribute "diagnosed_with". Similarly, for any procedures that exist for a patient, an edge is added from the patient node to each procedure node with the edge attribute "had_procedure". Furthermore, if a diagnosis has the same date as an encounter, then an edge is added between the corresponding diagnosis node and encounter node with the edge attribute "diagnosed_on".

At the step of extending 116, the graph-based model is extended with a set of one or more nodes of a clinical knowledge graph. The set of one or more nodes are connected to at least one node in the clinical knowledge graph which matches at least one node in the graph-based model.

As shown in Figure 6C and described in more detail below, the graph-based model can be enriched with relevant clinical knowledge extracted from a clinical knowledge graph. This allows new insights to be gained and new relationships and/or issues to be identified. This in turn can help improve patient outcomes whilst also providing a compact and efficient means of extending the clinical data associated with the document.

A clinical knowledge graph is a knowledge graph which semantically models clinical data and knowledge. Example clinical knowledge graphs include diagnostic, pharmacological, clinical best practice, care management, and care quality measurement knowledge graphs as are known in the art. Example third party clinical knowledge graphs include Google Health Knowledge Graph and Elsevier ClinicalKey. Other sources of such clinical knowledge include SNOMED CT, ICD-10, UMLS, RxNorm, and DrugBank.

To extend the graph-based model using the knowledge graph, at least one node within the clinical knowledge graph is identified which matches at least one node within the graph-based model. The two nodes can be matched based on a similarity between the two nodes satisfying a similarity criterion. At least one node in the clinical knowledge graph and at least one node in the graph-based model satisfy the similarity criterion if they are both linked to a common clinical code (e.g., both nodes represent, or are associated with, the same SNOMED code). Additionally, or alternatively, the two nodes can be matched based on a text-based similarity between one or more attributes of the two nodes exceeding a predefined threshold.

Once a set of matching nodes have been identified within the knowledge graph, the neighbouring nodes and outgoing/incoming edges of each matching node are identified within the knowledge graph. Any neighbouring nodes and/or edges within the knowledge graph which are not present within the graph-based model represent potential clinical knowledge or information which is missing from the structured set of clinical information. The graph-based model is thus extended by adding the neighbouring nodes and/or edges from the clinical knowledge graph to the graph-based model.

An example of extending a graph-based model with new knowledge obtained from a clinical knowledge graph is shown in Figures 6A and 6C as described below.

At the step of updating 118, the structured set of clinical information is updated based on data linked to the one or more nodes of the clinical knowledge graph.

As stated above, any neighbouring nodes within the knowledge graph which are not present within the graph-based model represent potential clinical knowledge or information which is missing from the structured set of clinical information. This missing clinical knowledge or information can thus be incorporated into the structured set of clinical information to extend and enrich the data and improve downstream tasks which utilise such data. In addition, the knowledge graph may include a missing relationship (i.e., edge) between two nodes within the graph-based model. For example, a neighbouring node which is not within the graph-based model may be connected in the knowledge graph to a node which matches with a node within the graph-based model. In such an example, both the neighbouring node and the edge can be added to the graph-based model. As a further example, an edge (relationship) between two nodes in the graph-based model may be added to the graph-based model based on the identification of the edge in the knowledge graph.

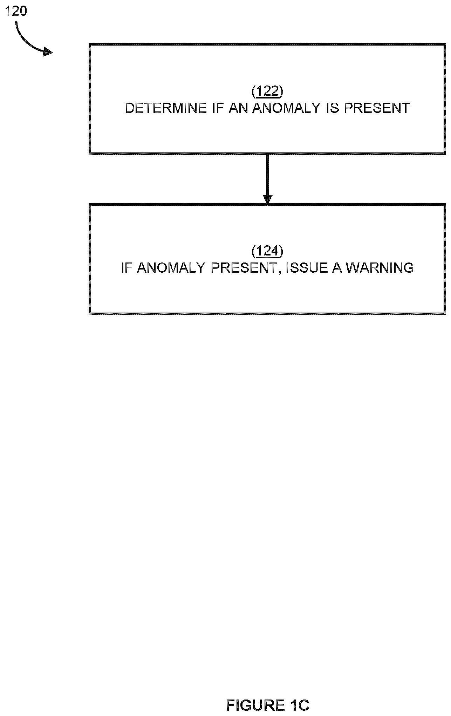

Figure 1C shows a sequence of steps 120 for determining anomalies within a structured set of clinical information according to an embodiment of the present disclosure.

The sequence of steps 120 comprises determining 122 if an anomaly is present and, if an anomaly is present, issuing 124 a warning. In one embodiment, the structured set of clinical information is generated by the method 100 shown in Figure 1A. The sequence of steps 120 can be performed as part of the steps described in relation to Figure 1A above (e.g., by performing the sequence of steps 120 after executing 108 the executable coding graph to generate the structured set of clinical information).

At the step of determining 122, a determination is made to ascertain if at least one anomaly is present within the structured set of clinical information based on an anomaly detection model for the clinical context.

In general, an anomaly may be considered to refer to an unexpected code and/or value within the structured set of clinical information. The code and/or value can be unexpected in relation to the clinical context of the document. For example, a code related to cervical screening is anomalous, or unexpected, when appearing in a document related to a prostate exam performed on a patient; whereas the same code would not be anomalous when appearing in a document reporting screening results to a patient. Therefore, the determination of an anomaly can be dependent upon the clinical context of the document.

The anomaly detection model determines that at least one anomaly is present within the structured set of clinical information if the structured set of clinical information comprises one or more of: a code which is not related to the clinical context; a value outside of an expected range of values; a transcription error; a repeated code; a non-clinical code; incorrect values; a missing expected code; and/ or a missing expected value. Additional anomalies detected by the anomaly detection model include units-based anomalies (e.g., a value is reported using units which do not correspond to the requisite reference units), date-based anomalies (e.g., a date is identified as being too far in the future or too far in the past), and workflow assignment anomalies (e.g., when a document is assigned an incorrect clinical class, such as a document associated with a medication change being assigned to a filing clinical class rather than a clinical class associated with a review by a medical practitioner). The anomaly detection model can be a rule-based model, a predictive model, or a set of one or more functions related to one or more anomalies being detected.

An anomaly corresponding to a code which is not related to the clinical context corresponds to a code (e.g., a SNOMED code) appearing within the structured set of clinical information which is not related to the clinical context of the document. Such an anomaly can be determined using a look-up-table of codes (or disallowed/unallowable codes) for each clinical context, a predictive model, or a knowledge graph (e.g., if a code is associated with a node which is more than a predetermined number of nodes/edges/connections away from a node related to the clinical context, then it is deemed anomalous).

An anomaly corresponding to a value outside of an expected range of values corresponds to a value extracted from the document and represented within the structured set of clinical information which is anomalous or an outlier. That is, by comparing a value for an attribute or property (e.g., blood test result, heart rate, cholesterol level, etc.) to an expected range of values for that attribute or property it can be determined whether the value is anomalous. Each specific attribute or property-e.g., age, height, weight, test results, etc.-can have an expected range of values which can be represented as either a maximum and minimum value or as a distribution. If a value lies outside of its corresponding range or values, or is an outlier with respect to the distribution, then it can be deemed anomalous.

An anomaly corresponding to a transcription error corresponds to a value and/or code which is incorrect due to an error in transcription. For example, a height value being reported in kilograms or a date reported as 31 February 1421. Such anomalies occur as a result of transcription of the document and/or as a result of the conversion of the document into a text-based representation. Transcription errors can be identified using predictive or rules-based models.

An anomaly corresponding to a repeated code occurs when the same code appears twice within the structured set of clinical information. For example, this may occur if the same diagnosis or medical test is referred to twice in a document but should only be recorded once in the structured set of clinical information.

An anomaly corresponding to a non-clinical code corresponds to a code being included within the structured set of clinical information which does not have a specific clinical context. For example, a quality and outcome framework (QOF) code. A non-clinical code anomaly can be detected using a look-up-table of non-clinical codes or using a predictive model/natural language processing approach to classify a code and/or its description as either clinical or non-clinical.

A missing expected code/value anomaly occurswhen a code or valuewhich is expected to be present within the structured set of clinical information is missing. For example, a structured set of clinical information not containing any blood test values or codes when the document from which it was generated relates to reporting blood test results. A missing expected code/value can be determined using a rule-based model (e.g., each clinical context, clinical domain, or document type has a list of expected code/values which are used as a checklist) or an inference model (e.g., using a large language model and/or a knowledge graph to infer that a code/value should be present but is not).

At the step of issuing 124, by the one or more processors, a warning related to the at least one anomaly is issued if at least one anomaly is present within the structured set of clinical information.

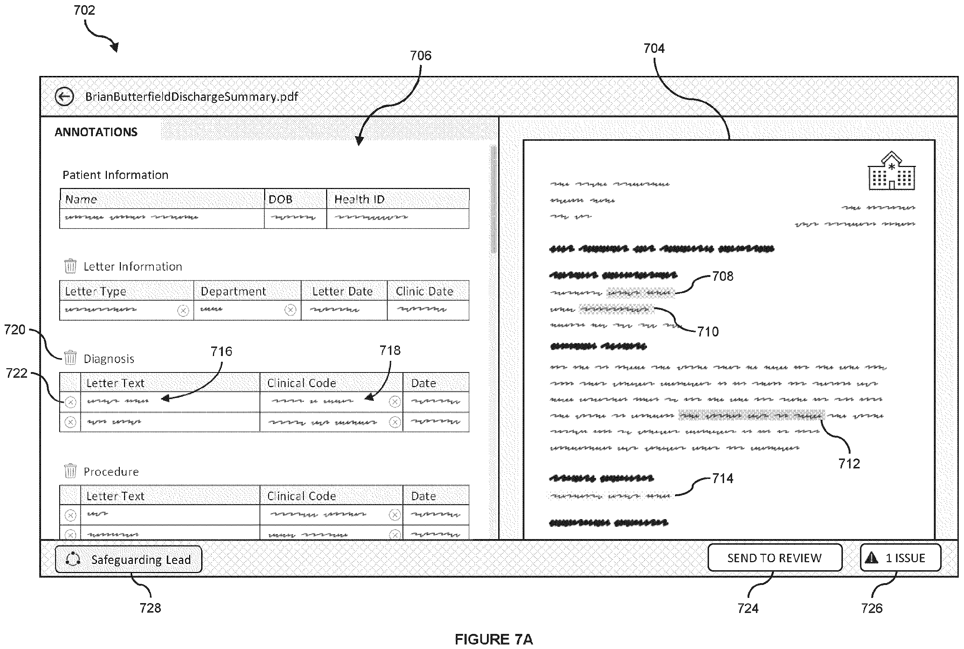

The warning can be displayed on a user interface viewable by a user (e.g., the user interface 702 shown in Figure 7A). Additionally, or alternatively, the warning can be transmitted to a monitoring and/or management system to flag that an anomaly has been identified.

In one embodiment, if at least one anomaly is present within the structured set of clinical information, the document and the structured set of clinical information are transmitted to a reviewing system where a human user can review the at least one anomaly and adjust the structured set of clinical information accordingly.

Additionally, or alternatively, the structured set of clinical information is updated based on the at least one anomaly. For example, the data in the structured set of clinical information related to the anomaly can be deleted from the structured set of clinical information or a predictive model can be used to predict a correct code or value in place of the anomalous code or value.

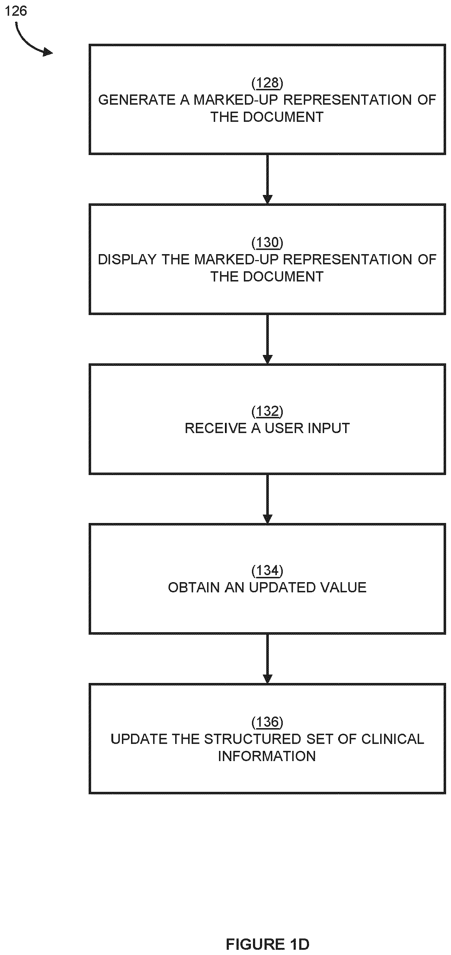

Figure 1D shows a sequence of steps 126 for generating, and interacting with, a marked up representation of a clinical document according to an embodiment of the present disclosure.

The sequence of steps 126 comprises generating 128 a marked-up representation of the document, displaying 130 the marked-up representation of the document, receiving 132 a user input, obtaining 134 an updated value, and updating 136 the set of clinical information. In one embodiment, the structured set of clinical information is generated for the document by the method 100 shown in Figure 1A (e.g., the sequence of steps 126 is performed after executing 108 the executable coding graph to generate the structured set of clinical information).

At the step of generating 128, a marked-up representation of the document is generated based on the structured set of clinical information.

Here, a marked-up representation of the document is to be understood as a version, copy, or representation of the document with portions of the document being styled (rendered or formatted) in a style or format that is not present within the document. For example, a portion of text within the document may be highlighted in red in the marked-up representation of the document. Alternatively, a portion of text within the document may be redacted or removed within the marked-up representation. The portions of the marked-up representation which are styled correspond to the portions of the document which are identified as relating to clinical data extracted from the document and appearing within the structured set of clinical information.

As such, a text portion within the marked-up representation of the document which is related to a datum of the structured set of clinical information is rendered according to a style linked to a semantic class of the datum. Each semantic class (type, clinical class, or category) is associated with a rendering style such as a highlight colour, a font colour, a font style, a border shape, a border style, a border colour, or the like. For example, all text portions corresponding to patient information may be displayed with a yellow highlight or background whilst all text portions corresponding to diagnoses may be displayed with a red highlight or background. As such, data from each semantic class is rendered consistently within the marked-up representation of the document. This allows a user to identify quickly and efficiently data which has been extracted from the document for each class or category.

At the step of displaying 130, the marked-up representation of the document is displayed within a user interface viewable by a user. Each rendered text portion is displayed as a selectable element in the user interface.

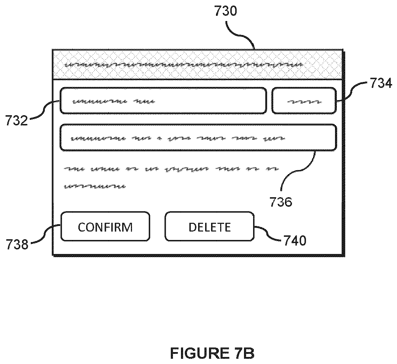

As shown in Figure 7B and described in more detail below, the structured set of clinical information can be concurrently displayed with the marked-up representation of the document within the user interface. Each element of the structured set of clinical information is displayed as a selectable element in the user interface. As such, a user is able to select either selectable elements linked to the data within the structured set of clinical information or selectable elements linked to the source information (text portion) within the document.

Additionally, or alternatively, a copy of the marked-up representation of the document can be saved to a persistent storage location.

At the step of receiving 132, a user input is received. The user input is associated with a first selectable element corresponding to a first rendered text portion related to a first datum of the structured set of clinical information. Alternatively, the user input is associated with a selectable element corresponding to the first data displayed within the structured set of clinical information.

At the step of obtaining 134, an updated value for the first datum from a user is obtained. That is, in consequence of the user input being received, the user provides an updated value for the first datum.

For example, a user interface such as that shown in Figure 7B and described below is provided to the user in response to the user input being received. The user interface allows the user to provide an updated value for the first datum.

At the step of updating 136, the first datum in the structured set of clinical information is updated to the updated value.

Therefore, the user can update an element of the structured set of clinical information, such as a clinical code or value, by interacting in situ with the marked-up representation of the document. This provides an intuitive and efficient interface for a user to edit and modify structured clinical information within the context of the document from which the clinical information was extracted (rather than interacting with the clinical information out of context such as within a separate application such as a spreadsheet or database application).

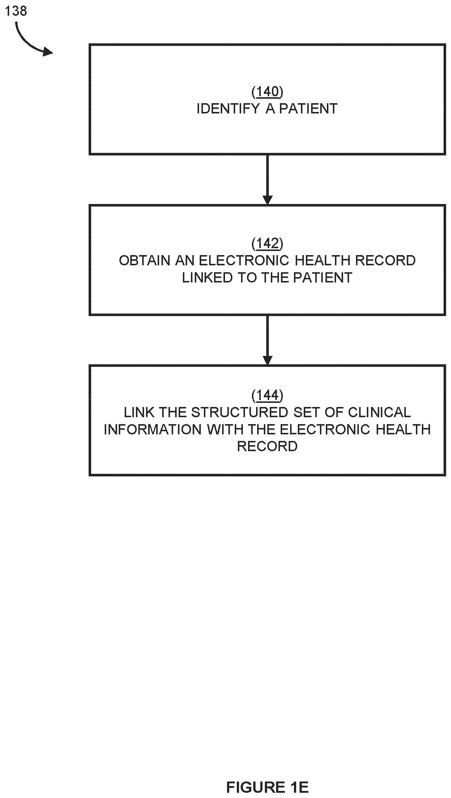

Figure 1E shows a sequence of steps 138 for linking patient data to a structured set of clinical information according to an embodiment of the present disclosure.

The sequence of steps 138 comprises identifying 140 a patient, obtaining 142 an electronic health record linked to the patient, and linking 144 the structured set of clinical information with the electronic health record. In one embodiment, the structured set of clinical information is generated by the method 100 shown in Figure 1A. The sequence of steps 138 can be performed as part of the steps described in relation to Figure 1A above (e.g., by performing the sequence of steps 138 after executing 108 the executable coding graph to generate the structured set of clinical information).

At the step of identifying 140, a patient referred to within the document is identified.

If the structured set of clinical information contains a patient identifier (e.g., a national health service number), then the patient identifier is used to identify the patient referred to within the document. Alternatively, if the document and/or the structured set of clinical information is linked to a patient object or a patient identifier, then the patient object or the patient identifier is used to identify the patient referred to within the document. For example, if the document or structured set of clinical information are obtained from a set of documents or data related to a specific patient, then the details relating to that patient can be used to identify the patient.

At the step of obtaining 142, an electronic health record (EHR) linked to the patient is obtained.

As is known, an EHR comprises the medical history of the patient and is stored/represented in electronic form (e.g., as a file on a device or system). The EHR can be identified from a database of EHRs by querying the database by a unique identifier for the patient (e.g., a national health service number). The EHR may be stored by a third party or health care provided and obtained via an application programming interface (API). In such instances, the EHR may comprise a reduced set of data or information relating to the patient for security purposes. The EHR can be obtained from the database or application programming interface (API) in an encrypted form and can be subsequently decrypted prior to being used.

At the step of linking 144, the structured set of clinical information is linked with one or more elements of the electronic health record.

Data or information within the EHR which are not present within the structured set of clinical information can be added to the structured set of clinical information to extend and enrich the data. In one embodiment, the structured set of clinical information is represented as a graph and the new information is added as one or more nodes or edges of the graph (as described in relation to Figure 6B below). Alternatively, the data within the patient's EHR which is not present within the structured set of clinical information is added to the relevant portions of the structured set of clinical information (e.g., adding a new medicament to the medication portion or sub-table of the structured set of clinical information and adding a new diagnosis to the diagnosis portion or sub-table of the structured set of clinical information). Additionally, or alternatively, data or information within the structured set of clinical information can be deleted and/or updated based on the data or information with the EHR. For example, if the structured set of clinical information contains data which is already present within the EHR, then this data can be deleted from the structured set of clinical information.

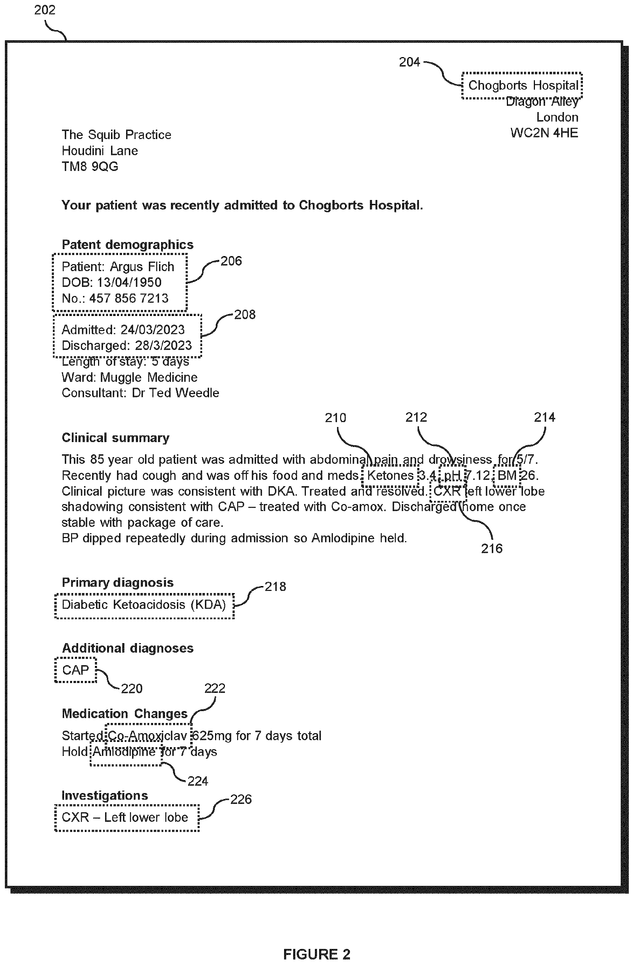

Figure 2 shows a document 202 having a clinical context according to an embodiment of the present disclosure.

The document 202 is a discharge summary of a patient's stay at a hospital. The document 202 comprises clinical information which can be extracted from the document 202 by a clinical knowledge extraction process (such as that described in relation to Figure 1A) and used to generate a structured set of clinical information. In particular, the document 202 comprises organisational information 204, patient information 206, and encounter information 208. The document 202 further comprises measurements 210, 212, 214 and procedures 216 and 226. The document 202 also comprises diagnoses 218, 220 and medications 222, 224.

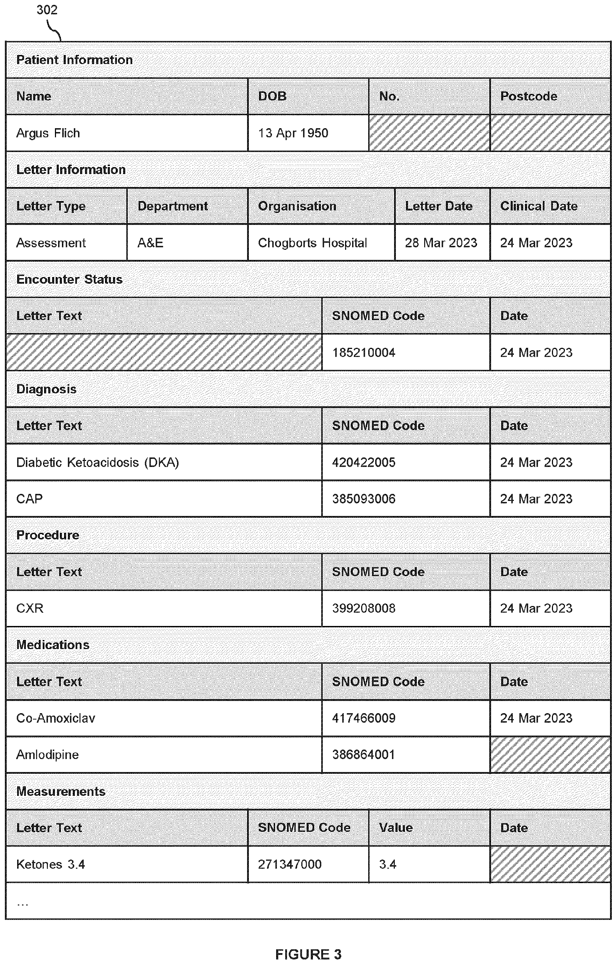

Figure 3 shows a structured set of clinical information 302 extracted from the document 202 shown in Figure 2 according to an embodiment of the present disclosure. The skilled person will appreciate that not all elements extracted from the document 202 of Figure 2 are shown in Figure 3 for brevity.

Figure 4A shows a portion 402 of an executable coding graph according to an embodiment of the present disclosure.

The portion 402 of the executable coding graph comprises a first branch node 404, a second branch node 406, and a third branch node 408. The portion 402 of the executable coding graph further comprises a first coding node 410 and a second coding node 412. Figure 4A further shows an exit point 414 and a text-based representation 416 of a document having a clinical context. The skilled person will appreciate that the exit point 414 is a convenience representing the exit point of the portion 402 of the executable coding graph and may correspond to a further node of the executable coding graph, a further processing function, a termination of execution of the executable coding graph, or the like.

The portion 402 of the executable coding graph is structured as a directed acyclic graph (DAG) of nodes which comprise processing logic for semantically analysing the text-based representation 416 of a document to extract clinical data related to medical procedures which is subsequently added to a structured set of clinical information. As will be described in more detail below, an executable coding graph is composed of branch nodes-such as the first branch node 404-interconnected with coding nodes-such as the first coding node 410.

The first branch node 404 semantically analyses the text-based representation 416 to evaluate a query related to the clinical context of the document. In the example of Figure 4A, the first branch node 404 comprises processing logic which determines if the text-based representation 416 contains information related to a clinical procedure such as an ophthalmic examination.

In general, a branch node (alternatively referred to as a conditional node or an extraction node) comprises processing logic which semantically analyses a text-based representation of a document to evaluate a query related to the clinical context of the document. For example, the first branch node 404 semantically analyses the text-based representation 416 to evaluate the query "does the document mention any medical procedures?". As such, the query is linked to, or forms a part of, the branch node. The evaluation of the query can then be used to determine which node connected to the branch node is to be executed next. Continuing the previous example, if mention of a clinical procedure is made, then the second branch node 406 is executed, else the process proceeds to the exit point 414. As such, a plurality of branch nodes may be coupled together to form a network, or sub-graph, of branch nodes and the DAG can comprise one or more networks, or sub-graphs, of branch nodes. In the example shown in Figure 4A, the first branch node 404, the second branch node 406, and the third branch node 408 form a network of branch nodes.

The processing logic of a branch node for evaluating the query linked to the branch node can be a natural language processing function. In general, the natural language processing function uses one or more natural language processing operations to analyse a portion, or all, of a text-based presentation of a document. For example, the processing logic may include a regular expression (regex) for identifying a text pattern within the text-based representation such as a date of birth or a numerical string of a given length (e.g., a medical identifier). The processing logic of the natural language processing function can form a part of the branch node or the branch node can include a reference to the natural language processing function. For example, the branch node can include the name of a natural language processing function within a library which is to be called when the branch node is executed. The library can be an external, or third party, library such that the name of the natural language processing function within the branch node is a reference to an endpoint or function of an application programming interface (API) of the external, or third party, library.

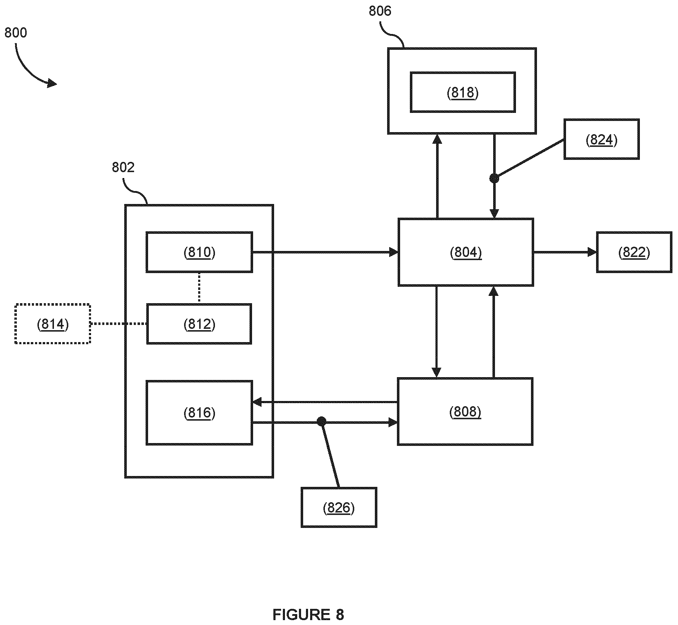

Additionally, or alternatively, the processing logic of a branch node for evaluating the query linked to the branch node can include the use of a large language model (LLM). That is, a prompt is provided to the LLM to perform the semantic analysis and determine the evaluation of the query. The prompt comprises a predefined command portion and a context portion which comprises at least a part of a text-based representation of a document. The predefined command portion comprises a command or instruction operable to cause the LLM to determine the evaluation of the query based on the context portion. For example, to determine if the document contains a reference to a medical procedure the predefined command portion may include an instruction such as "The following text is a clinical letter. Your job is to evaluate whether the following text refers to a medical procedure. For each medical procedure referred to within the text, provide the portion of the text which relates to the medical procedure". When using an LLM, the call to the LLM can be wrapped within a function or code block which is executed by the branch node to evaluate the query. The LLM can be internally deployed within the system in which the executable coding graph is executed (e.g., the system 800 shown in Figure 8). Alternatively, the LLM can be offered as a third party service. Example third party services include GPT-4o provided by OpenAI, Bloom by BigScience, and Google Bard.

If, as a result of executing the first branch node 404, it is determined that the document does not mention a medical procedure, then execution proceeds to the exit point 414. Otherwise, execution proceeds to the second branch node 406 where a semantic analysis of the text-based representation 416 is performed to determine if the medical procedure is an eye examination. That is, the second branch node 406 comprises processing or decision logic (e.g., a natural language processing function or a function using an LLM, as described above) operable to semantically analyse the text-based representation 416 to evaluate a query related to whether the medical procedure is an eye examination.

If, as a result of executing the second branch node 406, it is determined that the medical procedure is an eye examination, then the first coding node 410 is executed.

In general, a coding node comprises processing logic which assigns a clinical datum to the structured set of clinical information. The clinical datum can be determined based on a semantic analysis of the text-based representation 416 performed by the coding node and/or the semantic analysis performed by one or more prior branch nodes within the DAG. In one embodiment, a coding node is immediately preceded within the DAG by a network of branch nodes interconnected with the coding node. The network of branch nodes can be understood as defining the conditions in which the coding node is executed and thus the conditions in which the associated clinical datum is added to the structured set of clinical information.

A coding node therefore populates the structured set of clinical information with data extracted or inferred from the text-based representation 416 of the document. For example, a coding node may be preceded by a network of branch nodes which are used to determine if the document contains a confirmed diagnosis which is not present within the patient's history, if it is determined that the document does contain a confirmed diagnosis not present in the patient's history then the coding node is executed thereby causing a clinical datum linked to the diagnosis (e.g., a SNOMED code) to be added to the structured set of clinical information. As a further example, a coding node may include processing logic to extract the letter date from the text-based representation 416 of the document and is thus independent of any prior branch nodes (i.e., execution of the coding node is not conditional on the execution of a network of prior branch nodes).

The processing logic of a coding node can be a natural language processing function and/or involve the use of a large language model (LLM). The skilled person will appreciate that the above discussion of these functions in relation to branch nodes applies equally to the processing logic of a coding node. However, a key distinction is that a coding node includes processing logic to add a clinical datum to the structured set of clinical information. The clinical datum can be a value and/or a predefined code.

When the clinical datum added to the structured set of clinical information is a value, the value is extracted from the text-based representation 416 of the document by the coding node and/or one or more branch nodes within a prior network of branch nodes. Example values include a date of birth, a letter date, a patient identifier, or the like.

When the clinical datum added to the structured set of clinical information is a predefined code, the predefined code can be linked to the coding node (i.e., the coding node comprises the predefined code). That is, whenever the coding node is executed, the same predefined code (defined by the coding node) is added to the structured set of clinical information. For example, whenever a coding node is executed, the coding node may cause a predefined code corresponding to a certain medication to be added to the structured set of clinical information. Alternatively, the predefined code can be derived or defined from the decision logic executed as a result of executing a prior sequence of branch nodes connected to the coding node. That is, a branch node may determine that a first predefined code should be used if a first condition is met and a second predefined code used if a second condition is met such that the coding node adds either the first or the second predefined code to the structured set of clinical information depending on which condition is met. For example, a branch node may determine from the context of the document that the document originates in the United Kingdom and so a UK specific predefined code should be used whereas if the document originated in the United States, then a US specific predefined code would be used. The predefined coding node is one of: a Systematized Nomenclature of Medicine Clinical Terms (SNOMED CT) code; an International Classification of Disease (ICD)-9 code; an ICD-10 code; an ICD-11 code; a Healthcare Common Procedure Coding System (HCPCS) code; a Current Procedure Terminology (CPT) code; a medical prescription normalised Medical prescription (RxNorm) code; a Logical Observation Identifiers Names and Codes (LOINC) code; a Medical Subject Headings (MeSH) code; or a Unified Medical Language System (ULMS) code.

In the example shown in Figure 4A, the first coding node 410 includes processing logic for extracting the data related to the eye examination from the text-based representation 416. The first coding node 410 further includes processing logic for determining a predefined code related to an eye examination (e.g., SMOMED CT code 36228007). Both the code and the value are added as a clinical datum to the structured set of clinical information.

The third branch node 408 is executed if, as a result of executing the second branch node 406, it is determined that the medical procedure is not an eye examination. The third branch node 408 performs a semantic analysis of the text-based representation 416 to determine if the medical procedure is a biopsy. That is, the third branch node 408 comprises processing or decision logic (e.g., a natural language processing function or a function using an LLM, as described above) operable to semantically analyse the text-based representation 416 to evaluate a query related to whether the medical procedure is a biopsy.

If it is determined, as a result of executing the third branch node 408, that the medical procedure is not a biopsy, then the process proceeds to the exit point 414. Otherwise, the second coding node 412 is executed. The second coding node 412 includes processing logic for extracting data related to the biopsy procedure from the text-based representation 416 and adding this data to the structured set of clinical information.

The portion 402 shown in Figure 4A represents a sub-graph of a larger executable coding graph for extracting clinical knowledge from a document. In such embodiments, sub-graphs such as the portion 402 are arranged as processes within an executable coding graph.

Figure 4B shows an executable coding graph 418 according to an embodiment of the present disclosure.

The executable coding graph 418 comprises a first process 420, a second process 422, and a third process 424 arranged within a first parallel stage 426. The executable coding graph 418 further comprises a fourth process 428 arranged within a first sequential stage and a fifth process 430, a sixth process 432, a seventh process 434, and an eight process 436 arranged within a second parallel stage. The executable coding graph 418 also comprises a ninth process 438 and a tenth process 440 arranged within a second sequential stage 442.

The executable coding graph 418 shown in Figure 4B is operable to extract, from a text-based representation 444 of a document having a clinical context, a structured set of clinical information 446. The clinical context of the document is ophthalmology such that the executable coding graph 418 comprises processing logic for semantically analysing the text-based representation 444 of the document according to the ophthalmological context to extract ophthalmology data, and other clinical data, from the text-based representation 444.

As shown in Figure 4B, nodes within the executable coding graph 418 can be grouped to form one or more processes. A process can be understood as a higher-order structure comprising one or more nodes which form a sub-graph of the DAG (e.g., the portion 402 shown in Figure 4A). The node(s) within a process comprise processing logic for extracting information related to a single clinical concept or item of information from the text-based representation 444 such that the process comprises the operations (nodes) necessary for identifying and extracting clinical data related to the clinical concept or item of information from the text-based representation 444. For example, the fifth process 430 comprises the portion 402 shown in Figure 4A and so comprises the branch nodes and coding nodes related to medical procedures mentioned within the text-based representation 444 of the document.

In one embodiment, a process comprises at least one coding node for assigning a clinical datum (e.g., a code or value) to the structured set of clinical information 446.

Processes within the executable coding graph 418 are conceptually grouped into stages. A stage (alternatively referred to as a layer) is either parallel or sequential. Processes within a parallel stage are executed in parallel whilst processes in a sequential stage are executed sequentially. For example, the nodes within the first process 420, the second process 422, and the third process 424 of the first parallel stage 426 are executed concurrently, or near concurrently, whilst the nodes within the ninth process 438 of the second sequential stage 442 are executed prior to the nodes within the tenth process 440 of the second sequential stage 442. The processes within a stage or layer can be functionally or semantically related. For example, the processes within the first parallel stage 426 all relate to pre-processing operations whilst the processes within the second sequential stage 442 all relate to post-processing operations.

As stated above, the executable coding graph 418 is linked to the clinical context of ophthalmology and is operable to extract relevant clinical data from the text-based representation 444 of a document which shares the same clinical context (e.g., an ophthalmology referral letter).

The processes within the first parallel stage 426 are pre-processing processes: the first process 420 comprises nodes operable to extract dates from the text-based representation 444 of the document (e.g., letter date, clinic date, etc.); the second process 422 comprises nodes operable to extract a patient's medical identifier from the text-based representation 444 of the document (e.g., a National Health Service (NHS) number); and the third process 424 comprises nodes operable to extract patient information from the text-based representation 444 of the document (e.g., patient name, age, postcode, etc.).

The fourth process 428 within the first sequential stage is executed after all the processes within the first parallel stage 426 have been executed. Alternatively, the fourth process 428 is executed after at least the first process 420 of the first parallel stage 426 has been executed. The fourth process 428 comprises a branch node operable to determine if at least one date has been extracted from the text-based representation 444 of the document. If it is determined that at least one date has been extracted, then the processes within the second parallel stage are executed; otherwise, execution of the executable coding graph 418 is terminated or paused until a code can be identified (e.g., by a user).

The processes within the second parallel stage are directed to extracting ophthalmology specific information/data from the text-based representation 444 of the document. The fifth process 430 comprises nodes operable to extract data related to medical procedures from the text-based representation 444 of the document (e.g., the fifth process 430 comprises the portion 402 shown in Figure 4A). The sixth process 432 comprises nodes operable to extract data related to referrals from the text-based representation 444 of the document (e.g., GOS18 referrals). The seventh process 434 comprises nodes operable to extract data related to encounters mentioned within the text-based representation 444 of the document (e.g., an appointment with a medical professional, an eye examination with an ophthalmologist, etc.). The eight process 436 comprises nodes operable to extract data related to diagnoses mentioned within the text-based representation 444 of the document (e.g., new diagnoses, existing diagnoses mentioned within the patient's medical history, etc.).

The ninth process 438 of the second sequential stage 442 is executed after all processes within the second parallel stage have been executed. Alternatively, the ninth process 438 is executed after at least one of the processes within the second parallel stage has been executed. The ninth process 438 comprises nodes which perform post-processing operations to sanitise the structured set of clinical information 446 (e.g., removing duplicates, removing specific codes known not to be relevant to the clinical context, etc.). The tenth process 440 of the second sequential stage 442 is executed after the ninth process 438 has been executed and comprises nodes operable to execute a clinical class model on the structured set of clinical information 446 (as will be described in more detail in relation to Figure 5 below).

The structured set of clinical information 446 therefore corresponds to a structured representation of the data contained within the text-based representation 444 of the document which has been automatically extracted via execution of the executable coding graph 418.

The skilled person will appreciate that the use of ophthalmology as a clinical context is illustrative and is in no way intended to limit the scope of the present disclosure and a plurality of executable coding graphs can be used to extract relevant clinical data from a range of different clinical contexts.

Figure 5 shows a clinical class model 502 according to an embodiment of the present disclosure.

The clinical class model 502 comprises a sequence of clinical gates including a first clinical gate 504 linked to a first clinical class 506, a second clinical gate 508 linked to a second clinical class 510, a third clinical gate 512 linked to a third clinical class 514, and a fourth clinical gate 516 linked to a fourth clinical class 518. Figure 5 further shows a structured set of clinical information 520 provided to the clinical class model 502.

The clinical class model 502 is a rules-based model for automatically determining, from the structured set of clinical information 520 extracted from a document, a recipient entity to which the document should be transmitted. That is, the clinical class model 502 analyses the structured set of clinical information 520 to determine a clinical class to assign to the document and thus where the document should be transmitted/forwarded/sent. In embodiments, a clinical class model such as the clinical class model 502 is incorporated into an executable coding graph such that the document is automatically transmitted to the relevant recipient entity after the clinical information has been extracted. This is shown by the operations of the tenth process 440 of the executable coding graph 418 of Figure 4B.

The clinical gates of the clinical class model 502 are organised in order of descending clinical risk. That is, the first clinical gate 504 applies criteria associated with the highest clinical risk, whilst the fourth clinical gate 516 applies criteria associated with the lowest clinical risk. In this way, the clinical class model502 is optimized for clinical risk such that documents having a high-clinical risk are handled first. Moreover, the structure of the clinical class model 502 is such that the risk associated with misclassifying documents is mitigated.

A clinical class, such as the first clinical class 506, is a label, group, tag, class, or identity assigned to the document by the clinical class model 502. A clinical class is associated, or linked, with at least one recipient entity. Here, a recipient entity is a system, storage location, or individual to which the document is to be transmitted. A recipient entity is linked with a communication channel along which the document is transmitted (e.g., email, file transfer protocol, secure communication channel, persistent storage, etc.).

The first clinical gate 504 applies a first criterion to the structured set of clinical information 520 to determine if the document is to be assigned to the first clinical class 506. That is, if the first criterion is satisfied, then the document is assigned to the first clinical class 506 and the document is transmitted to the recipient entity (or entities) associated with the first clinical class 506. In one embodiment, if the first criterion is satisfied, then the document is also assigned to the second clinical class 510 and the document is also transmitted to the recipient entity (or entities) associated with the second clinical class 510.

The first clinical gate 504 relates to safeguarding issues and the first criterion is satisfied if the structured set of clinical information 520 contains a safeguarding concern or issue. For example, the first criterion may be evaluated on the basis of whether the structured set of clinical information 520 contains SNOMED code 371772001 (domestic violence) or SNOMED code 82313006 (suicide attempt). The first clinical class 506 is associated with a safeguarding concern and the associated entity is a safeguarding lead and, in consequence of the first criterion being met, the document is automatically transmitted via email, secure communication channel, internal messaging channel, etc. to the safeguarding lead. In one embodiment, the document is also assigned to the second clinical class 510 if the first criterion is met. The second clinical class 510 is associated with a medical practitioner or service such as a general practitioners' office, hospital, or the like. Thus, in consequence of the first criterion being met, the document can also be automatically transmitted via email, secure communication channel, internal messaging channel, etc. to the relevant medical practitioner or service.

The first clinical gate 504 applies a first criterion to the structured set of clinical information 520 to determine if the document is to be assigned to the first clinical class 506. That is, if the first criterion is satisfied, then the document is assigned to the first clinical class 506 and the document is transmitted to the recipient entity (or entities) associated with the first clinical class 506. In one embodiment, if the first criterion is satisfied, then the document is also assigned to the second clinical class 510 and the document is also transmitted to the recipient entity (or entities) associated with the second clinical class 510.

If the first criterion applied by the first clinical gate 504 is met, then execution of the clinical class model 502 can terminate. Otherwise, execution proceeds to the second clinical gate 508.

The second clinical gate 508 applies a second criterion to the structured set of clinical information 520 to determine if the document is to be assigned to the second clinical class 510. That is, if the second criterion is satisfied, then the document is assigned to the second clinical class 510 and the document is transmitted to the recipient entity (or entities) associated with the second clinical class 510.

The second clinical gate 508 relates to general medical issues and the second criterion is satisfied if the structured set of clinical information 520 contains at least one clinical issue. Examples of clinical issues include any safeguarding issue(s), a diagnosis, a medication change, a referral, a rejected referral, an emergency admission discharge summary, a police, court, or social worker letter, and the like. The second clinical class 510 is associated with a general medical or clinical issue and the associated entity is a medical or clinical practitioner or entity such as a doctor or medical team within a doctor's surgery, hospital, or medical institution. In consequence of the second criterion being met, the document is automatically transmitted via email, secure communication channel, internal messaging channel, etc. to the associated entity.

In one embodiment, a message is sent to a recipient entity in consequence of the second criterion being met. For example, if a document contains information related to a medication change or repeat prescription, then a message may be transmitted to a pharmacist to authorise the medication change or repeat prescription.

If the second criterion applied by the second clinical gate 508 is met, then execution of the clinical class model 502 can terminate. Otherwise, execution proceeds to the third clinical gate 512. In one embodiment, the execution of the clinical class model 502 proceeds to the third clinical gate 512 when the second criterion applied by the second clinical gate 508 is met such that the clinical class model 502 operates sequentially or partially sequentially.

The third clinical gate 512 applies a third criterion to the structured set of clinical information 520 to determine if the document is to be assigned to the third clinical class 514. That is, if the third criterion is satisfied, then the document is assigned to the third clinical class 514 and the document is transmitted to the recipient entity (or entities) associated with the third clinical class 514.

The third clinical gate 512 relates to practice-based issues and the third criterion is satisfied if the structured set of clinical information 520 contains at least one practice issue. Examples of practice-based issues include a new address or change of address, a repeat test required, a follow-up appointment, a request for information, and the like. The third clinical class 514 is associated with a reception or admin entity or team within a doctor's surgery, hospital, or medical institution. In consequence of the third criterion being met, the document is automatically transmitted via email, secure communication channel, internal message channel, etc. to the associated entity.

In one embodiment, the document and/or a message is sent to a patient in consequence of the third criterion being met. For example, if a document contains information related to a follow-up appointment for a patient, then a message inviting the patient to book a follow up appointment may be transmitted to the patient (e.g., via e-mail, SMS, or the like).

If the third criterion applied by the third clinical gate 512 is met, then execution of the clinical class model 502 can terminate. Otherwise, execution proceeds to the fourth clinical gate 516.

The fourth clinical gate 516 relates to any other issues not covered by the previous gates and the fourth criterion is a default criterion (i.e., the criterion is met by default). Examples of other issues include an assessment or report, a dental letter, an appointment letter, a screening test, and the like. The fourth clinical class 518 is associated with a filing or storage entity within a doctor's surgery, hospital, or medical institution. In consequence of the fourth criterion being met, the document is automatically stored at a storage location linked to the filing or storage entity and/or is transmitted via email, secure communication channel, internal message channel, etc. to the filing or storage entity.

Figure 5 illustrates an example of clinical class model which models a specific workflow protocol and the skilled person will appreciate that other clinical class models and workflow protocols can be used. For example, other clinical classes may exist for performance of tasks such as filing of lab results and/or obfuscation of documents. Moreover, a document may be decomposed into a set of subtasks or sub-portions such that only the relevant portion of the document is transmitted to the recipient entity. For example, if a document contains information regarding a medication change and a set of lab results, the information regarding the medication change is sent to a first recipient entity (e.g., a pharmacist or associated medical practitioner) whilst the information regarding the set of lab results is sent to a second recipient entity. These can be sent either in parallel or sequentially.

Figures 6A-6C illustrate the incorporation of new knowledge into a graph-based representation of a set of structured clinical information according to embodiments of the present disclosure.

Figure 6A shows a graph 602A corresponding to an example graph-based model (or graph-based representation) generated from a structured set of clinical information extracted from a document (e.g., using the method 100 of Figure 1A). The graph 602A comprises a patient node 604, a procedure node 606, a first medical condition node 608, and a first medication node 610.

Whilst not shown, the document from which the structured set of clinical information is extracted (and the graph 602A is subsequently generated from) relates to an encounter between a patient and a general practitioner (GP). The document refers to a skin biopsy that was taken from the patient at the encounter. The document further refers to the patient being diagnosed with a depressive disorder and being prescribed Diazepam to treat the depressive disorder. For ease of reference, nodes and relationships relating to encounters are omitted from the example shown in Figures 6A-6C. The skilled person will appreciate that such information can be incorporated in the graph-based representation of a structured set of clinical information to enable auditing of diagnoses, prescriptions, etc.

The graph 602A shown in Figure 6A is generated from the structured set of clinical information using an approach as described in relation to Figure 1B. Each node within the graph 602A corresponds to a piece of clinical information or clinical data extracted from the document. The graph 602A is associated with a patient identified by the patient node 604. The document, and thus the clinical information extracted therefrom, is associated with the patient. The patient node 604 can comprise attributes related to the patient such as patient identifier, name, address, medical practice, etc. In addition, the patient node 604 can comprise attributes linking back to the document such as the portion of text, or the location, within the document from which the relevant data related to the patient-such as patient identifier, name, etc.-was identified and extracted.

The procedure node 606 corresponds to (i.e., represents, identifies, or is associated with) a procedure referred to in the document and represented within the structured set of clinical information. More particularly, the procedure node 606 corresponds to a skin biopsy performed on the patient and referred to in the document (and thus included in the structured set of clinical information). The node 606 can comprise attributes related to the procedure such as the SNOMED code for excisional skin biopsy (i.e., 1251630002), the date the procedure occurred, the date of the document, and the portion of text, or the location, within the document which referred to the procedure. The patient node 604 is linked to the procedure node 606 by an edge having the relationship "had_procedure" such that the graph 602A models the information found within the document that the patient has had an excisional skin biopsy.

The first medical condition node 608 corresponds to a medical condition referred to in the document and represented within the structured set of clinical information. More particularly, the first medical condition node 608 corresponds to a diagnosis of a depressive disorder referred to in the document. The first medical condition node 608 can comprise attributes related to the medical condition such as the SNOMED code for a depressive disorder (i.e., 35489007), the date the diagnosis was made, the date of the document, and the portion of text, or the location, within the document which referred to the medical condition. The patient node 604 is linked to the first medical condition node 608 by an edge having the relationship "diagnosed_with" such that the graph 602A models the information found within the document that the patient has been diagnosed with a depressive disorder.

The first medication node 610 corresponds to a medication referred to in the document and represented within the structured set of clinical information. More particularly, the first medication node 610 corresponds to a dosage of Diazepam which is referred to within the document as being given to the patient to treat the depressive disorder. The first medication node 610 can comprise attributes related to the medication such as the SNOMED code for Diazepam (i.e., 387264003), the dosage regimen, the date the medication was prescribed, the date of the document, and the portion of text, or the location, within the document which referred to the medication. The patient node 604 is linked to the first medication node 610 by an edge having the relationship "prescribed" such that the graph 602A models the information found within the document that the patient has been prescribed Diazepam. The first medication node 610 is linked to the first medical condition node 608 by an edge having the relationship "treats" such that the graph 602A models the information found within the document that the patient has been prescribed Diazepam to treats the depressive disorder.

Figure 6B shows the graph 602A of Figure 6A having been extended to generate a graph 602B according to embodiments of the present disclosure.

The graph 602B corresponds to the graph 602A described above with nodes added from the patient's electronic health record (EHR). More particularly, the graph 602A has been extended using the approach described in relation to Figure 1E above such that a second medical condition node 612 and a second medication node 614 are included in the graph 602B. Nodes which have been newly added to the graph are shaded within Figure 6B.

The second medical condition node 612 corresponds to a previously diagnosed medical condition-chronic pain-that is included within the patient's EHR. The second medical condition node 612 can comprise attributes related to the medical condition such as the SNOMED code for chronic pain (i.e., 82423001), the date the diagnosis was made, an indication that the condition was obtained from an EHR, etc. The patient node 604 is linked to the second medical condition node 612 by an edge having the relationship "diagnosed_with" (i.e., the patient has been diagnosed with chronic pain).

The second medication node 614 corresponds to a medication-Tramadol-which is referred to in the patient's EHR as being prescribed to treat the patient's chronic pain. The second medication node 614 can comprise attributes related to the medication such as the SNOMED code for Tramadol (i.e., 386858008), the dosage regimen, the date the medication was prescribed, , an indication that the condition was obtained from an EHR, etc. The patient node 604 is linked to the second medication node 614 by an edge having the relationship "prescribed" such that the graph 602B models the information found within the document that the patient has been prescribed Tramadol. The second medication node 614 is linked to the second medical condition node 612 by an edge having the relationship "treats" (i.e., Tramadol has been prescribed to the patient to treat chronic pain).

Figure 6C shows the graph 602B of Figure 6B having been extended to generate a graph 602C according to embodiments of the present disclosure.

The graph 602C corresponds to the graph 602B described above with elements added from a clinical knowledge graph. More particularly, the graph 602B has been extended using the approach described in relation to Figure 1B above such that a third medical condition node 616 and a new edge 618 are included in the graph 602C.

The third medical condition node 616 corresponds to the medical condition "Basal Cell Carcinoma" which is extracted from the knowledge graph via the common concept of excisional skin biopsy. That is, both the clinical knowledge graph and the graph 602B comprise a node representing excisional skin biopsy (e.g., both graphs include a node having the SNOMED code 1251630002). The excisional skin biopsy node within the clinical knowledge graph is connected to a node describing the purpose for which such biopsies are made-i.e., the knowledge graph comprises a node corresponding to the concept of Basal Cell Carcinoma and an edge connected between the two nodes representing the relationship "used_to_diagnose". As the graph 602B does not include this node or edge, they are included in the graph 602C such that the graph is now enriched with new information regarding the procedure referred to in the document (and represented by the procedure node 606).

The new edge 618 corresponds to a relationship which is extracted from the knowledge graph based on the common concepts of Diazepam and Tramadol. That is, both the clinical knowledge graph and the graph 602B comprise nodes representing Diazepam and Tramadol (e.g., both graphs include nodes having the SNOMED codes 387264003 and 386858008). In the clinical knowledge graph, the two nodes are connected by an edge representing the interaction between Diazepam and Tramadol. Specifically, the edge represents the fact that Diazepam and Tramadol can adversely interact and cause negative side effects. As the graph 602B does not include or model this knowledge-i.e., does not include an edge connecting the first medication node 610 and the second medication node 614-the new edge 618 is included in the graph 602C. Consequently, new information is incorporated within the graph 602C which can then be used to alert a user or medical practitioner that there may be an issue with the patient's medication.

Therefore, by transforming unstructured document data into a structured set of clinical information, data from different sources and systems can be integrated into a single coherent model thereby allowing data to be enriched and new insights to be obtained. This further enhances the interoperability of such systems by allowing unstructured data to be represented within a common structure.

Figure 7A shows a user interface 702 according to an embodiment of the present disclosure.

The user interface 702 includes a first area within which a marked-up representation 704 of a document is displayed and a second area within which a representation 706 of a structured set of clinical information linked to the document is displayed. The marked up representation 704 of the document comprises a first selectable element 708, a second selectable element 710, a third selectable element 712, and a fourth selectable element 714. The representation 706 of the structured set of clinical information comprises tabular information including a portion 716 of document text and a clinical code 718. The portion 716 of document text and the clinical code 718 are both associated with the first selectable element 708. The second area further includes a first selectable user interface (UI) element 720 and a second selectable UI element 722. The user interface 702 further includes a third selectable UI element 724, a warning indicator 726, and a recipient indicator 728.

The marked-up representation 704 of the document and the representation 706 of the structured set of clinical information are generated from a structured set of clinical information which is extracted from the document using a method such as the method 100 shown in Figure 1A. The marked-up representation 704 of the document is generated using a method such as that shown in Figure 1D.

The first selectable element 708, the second selectable element 710, the third selectable element 712, and the fourth selectable element 714 are all associated with a text portion of the marked-up representation 704 of the document. Each text portion is related to a datum extracted from the document and included within the structured set of clinical information. Each selectable element is rendered within the user interface 702 according to a style linked to a semantic class of the datum. As shown in Figure 7A, the first selectable element 708 and the second selectable element 710 are rendered in the same style as they are both associated with data related to diagnoses; whilst the third selectable element 712 and the fourth selectable element 714 are rendered in different styles as they are associated with data related to medications and procedures respectively.

Here, a style corresponds to the way a selectable element is presented or rendered within the user interface 702 to emphasize that it is associated with extracted clinical data and is selectable. A style can include aspects such as font, font weight, font style (e.g., bold, italic, etc.), font colour, highlight colour, bounding box shape, bounding box border style, and the like. Each class (e.g., patient information, encounter, procedure, etc.) of a structured set of clinical information has a corresponding style such that data and text within a document related to each semantic class can be quickly and efficiently identified within the user interface 702.

A selectable element within the user interface 702 is selectable by a user to initiate a process to review and/or modify the data associated with the selectable element. For example, and as described in more detail in relation to Figure 7B below, a user may select the first selectable element 708 to review and modify the clinical code and/or value associated with the text portion linked to the first selectable element 708. In one embodiment, the portion 716 of document text and the clinical code 718 are selectable elements such that selection of either of these elements allows a user to review and modify the associated clinical code and/or value associated.

The first selectable user interface (UI) element 720 and the second selectable UI element 722 are examples of UI elements which allow a user to remove or delete data from the structured set of clinical information. For example, selection of the first selectable UI element 720 results in all diagnoses within the structured set of clinical information being deleted whilst selection of the second selectable UI element results in a single diagnosis being deleted from the structured set of clinical information.

The third selectable UI element 724 allows a user to send the marked-up representation 704 of the document and the structured set of clinical information to a further entity or user for review. The warning indicator 726 provides visual feedback to the user that an issue has been detected within the structured set of clinical information. The issue is identified by an anomaly detection model which is specific to the clinical context of the document. Examples of issues within the structured set of clinical information include: a code which is not related to the clinical context; a value outside of an expected range of values; a transcription error; a repeated code; a non-clinical code; incorrect values; a missing expected code; and/ or a missing expected value. By selecting the warning indicator 726, the user is provided with an interface which allows them to review the warning and adjust the code, date, text, unit, and/or value causing the issue. For example, if an incorrect clinical code has been assigned, then the user can replace this clinical code with a correct clinical code. Alternatively, if an unexpected value has been encountered, then the user can confirm or reject this value.

The recipient indicator 728 provides an indication to the user as to the recipient of the document determined according to a clinical class model (as described in relation to Figure 5). In the example shown in Figure 7A, it can be seen that the document will be, or has been, transmitted to the safeguarding lead. The user can select the recipient indicator 728 to adjust the recipient (e.g., update the recipient from safeguarding lead to medical practitioner), view and/or adjust the clinical class assigned to the document, check on the status of the transmission (e.g., has the document been sent, has the document been received, has the document been logged, etc.), and the like.

Figure 7B shows a portion 730 of a user interface (UI) according to an embodiment of the present disclosure.

The portion 730 of the UI comprises a first text area 732, a second text area 734, a third text area 736, a confirmation UI element 738, and a deletion UI element 740. As described briefly in relation to Figure 7A above, the portion 730 of the UI is shown in consequence of a user selecting a selectable element in the user interface 702 of Figure 7A. That is, the portion 730 is shown when the user wants to initiate a process to review and/or modify the data associated with the selectable element (e.g., change the clinical code).

The first text area 732 provides a description of the clinical code associated with the selectable element, the second text area 734 indicates the clinical code associated with the selectable element, and the third text area 736 provides a brief description of the clinical code or an indication of the text within the document associated with the selectable element and the clinical code. In one embodiment, the user is able to change the code within the second text area 734 to adjust the clinical code associated with the selectable element. Once changed, the user can select the confirmation UI element 738 to confirm the change. Alternatively, the user is able to change other elements such as the text within the first text area 732 and/or the third text area 736. Selection of the deletion UI element 740 by the user results in the clinical code associated with the selectable element being deleted from the structured set of clinical information.

Whilst the above description of Figure 7B is made with reference to clinical codes, the skilled person will appreciate that the same interface and user interaction mechanism can be used to allow a user to review, modify, or delete a value associated with a selectable element (e.g., a blood test result, a patient name, etc.).

Figure 8 shows a system 800 according to an aspect of the present disclosure.

The system comprises a memory 802, an orchestration module 804, a document classification module 806, and a coding graph module 808. The memory 802 comprises a text-based representation 810 of a document 812 having a clinical context 814. The memory 802 further comprises a plurality of executable coding graphs 816. The document classification module 806 comprising a classifier 818 trained to output a predicted clinical identifier from text provided as input. The coding graph module 808 is configured to identify a respective executable coding graph from the plurality of executable coding graphs 820 based on a respective clinical context. The skilled person will appreciate that the system 800 is operable to perform any of the methods described above in relation to Figures 1A-1E.

The orchestration module 804 is configured to generate a structured set of clinical information 822 from the text-based representation 810 of the document 812. The text-based representation 810 of the document 812 is generated using a process such as optical character recognition (OCR) or using a multi-modal large language model (LLM). The document 812 has a clinical context 814 which refers to the context of the document 812 within the clinical or healthcare setting. The clinical context 814 is linked to a clinical domain of the document 812 and a type of the document 812.

The orchestration module 804 is configured to provide one or more portions of the text-based representation 810 of the document 812 to the document classification module 806 to determine an identifier 824 for the document 812. The identifier uniquely identifies the clinical context 814 of the document 812. The document classification module 806 implements one or more classifiers (e.g., machine learning models, predictive models, statistical models, etc.) which are configured or trained to determine, from a text-based portion of a document, an identifier to assign to the document.

The orchestration module 804 is further configured to provide the identifier 824 to the coding graph module 808 to identify an executable coding graph 826 for the clinical context 814. Each of the plurality of executable coding graphs 816 is indicative of a procedure for coding a clinical document according to a corresponding clinical context and comprises a network of branch nodes interconnected with a plurality of coding nodes thereby forming a directed acyclic graph. A branch node of the network of branch nodes is operable to determine which node connected to the branch node is to be executed next according to an evaluation of a query related to the corresponding clinical context and linked to the branch node. The query is evaluated based on a semantic analysis of the clinical document. A coding node of the plurality of coding nodes is operable to assign a clinical datum to a structured set of clinical information linked to the clinical document. The clinical datum can be determined from the semantic analysis performed by executing a prior sequence of branch nodes connected to the coding node.

The orchestration module 804 is further configured to execute the executable coding graph 826 on the text based representation 810 of the document 812 thereby generating the structured set of clinical information 822 linked to the document 812.

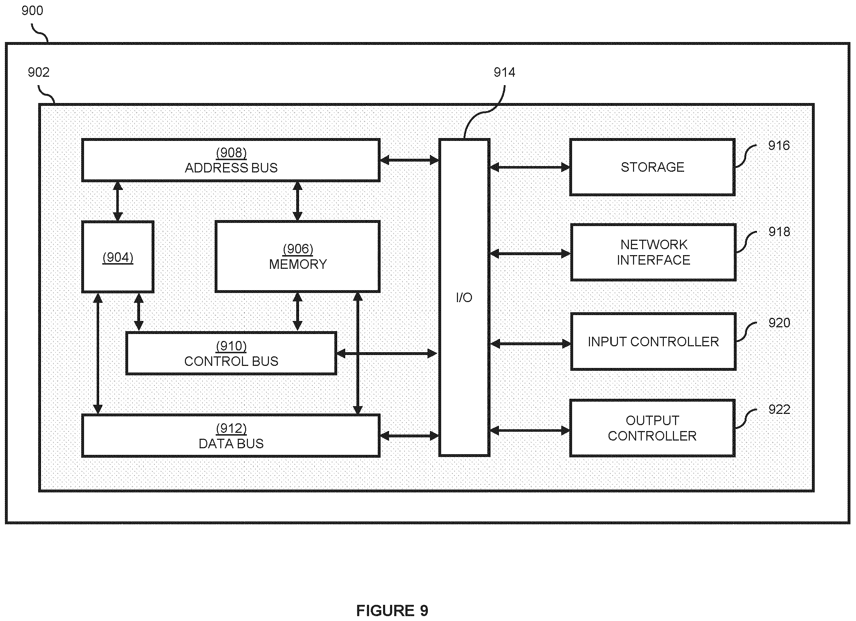

Figure 9 shows an example computing system for carrying out the methods of the present disclosure. Specifically, Figure 9 shows a block diagram of an embodiment of a computing system according to example embodiments of the present disclosure.

Computing system 900 can be configured to perform any of the operations disclosed herein such as, for example, any of the operations discussed with reference to the functional modules and units described in relation to Figures 2 to 8 or the steps discussed with reference to Figures 1A-1E. Computing system includes one or more computing device(s) 902. The one or more computing device(s) 902 of computing system 900 comprise one or more processors 904 and memory 906. One or more processors 904 can be any general purpose processor(s) configured to execute a set of instructions. For example, one or more processors 904 can be one or more general-purpose processors, one or more field programmable gate array (FPGA), and/or one or more application specific integrated circuits (ASIC). In one embodiment, one or more processors 904 include one processor. Alternatively, one or more processors 904 include a plurality of processors that are operatively connected. One or more processors 904 are communicatively coupled to memory 906 via address bus 908, control bus 910, and data bus 912. Memory 906 can be a random access memory (RAM), a read only memory (ROM), a persistent storage device such as a hard drive, an erasable programmable read only memory (EPROM), and/or the like. The one or more computing device(s) 902 further comprise I/O interface 914 communicatively coupled to address bus 908, control bus 910, and data bus 912.

Memory 906 can store information that can be accessed by one or more processors 904. For instance, memory 906 (e.g., one or more non-transitory computer-readable storage mediums, memory devices) can include computer-readable instructions (not shown) that can be executed by one or more processors 904. The computer-readable instructions can be software written in any suitable programming language or can be implemented in hardware. Additionally, or alternatively, the computer-readable instructions can be executed in logically and/or virtually separate threads on one or more processors 904. For example, memory 906 can store instructions (not shown) that when executed by one or more processors 904 cause one or more processors 904 to perform operations such as any of the operations and functions for which computing system 900 is configured, as described herein. In addition, or alternatively, memory 906 can store data (not shown) that can be obtained, received, accessed, written, manipulated, created, and/or stored. In some implementations, the one or more computing device(s) 902 can obtain from and/or store data in one or more memory device(s) that are remote from the computing system 900.

Computing system 900 further comprises storage unit 916, network interface 918, input controller 920, and output controller 922. Storage unit 916, network interface 918, input controller 920, and output controller 922 are communicatively coupled to the central control unit (i.e., the memory 906, the address bus 908, the control bus 910, and the data bus 912) via I/O interface 914.

Storage unit 916 is a computer readable medium, preferably a non-transitory computer readable medium, comprising one or more programs, the one or more programs comprising instructions which when executed by the one or more processors 904 cause computing system 900 to perform the method steps of the present disclosure. Alternatively, storage unit 916 is a transitory computer readable medium. Storage unit 916 can be a persistent storage device such as a hard drive, a cloud storage device, or any other appropriate storage device.

Network interface 918 can be a Wi-Fi module, a network interface card, a Bluetooth module, and/or any other suitable wired or wireless communication device. In an embodiment, network interface 918 is configured to connect to a network such as a local area network (LAN), or a wide area network (WAN), the Internet, or an intranet.

Although the invention has been described above with reference to one or more preferred embodiments, it will be appreciated that various changes or modifications can be made without departing from the scope of the invention as defined in the appended claims. The word "comprising" can mean "including" or "consisting of" and therefore does not exclude the presence of elements or steps other than those listed in any claim or the specification as a whole. The mere fact that certain measures are recited in mutually different dependent claims does not indicate that a combination of these measures cannot be used to advantage.

### NUMBERED STATEMENTS

1. A method for context-based clinical knowledge extraction, the method comprising:
   obtaining, by one or more processors, a text-based representation of a document having a clinical context;
   determining, by the one or more processors, an identifier which uniquely identifies the clinical context of the document by providing one or more portions of the text-based representation of the document to a classifier trained to generate a predicted identifier from text provided as input;
   identifying, by the one or more processors, an executable coding graph from a plurality of executable coding graphs based on the identifier of the clinical context, the executable coding graph indicative of a procedure for coding the document according to the clinical context and comprising a network of branch nodes interconnected with a plurality of coding nodes thereby forming a directed acyclic graph, wherein:
      a branch node of the network of branch nodes is operable to determine which node connected to the branch node is to be executed next according to an evaluation of a query related to the clinical context and linked to the branch node, wherein the query is evaluated based on a semantic analysis of the text-based representation of the document; and
      a coding node of the plurality of coding nodes is operable to assign a clinical datum to a structured set of clinical information linked to the document,
      wherein the clinical datum is determined from the semantic analysis performed by executing a prior sequence of branch nodes connected to the coding
      node; and
   executing, by the one or more processors, the executable coding graph on the text-based representation of the document thereby generating the structured set of clinical information linked to the document.
2. The method of statement 1 further comprising:
   transmitting, by the one or more processors and along a communication channel, the document to a recipient entity associated with a clinical class identified from the structured set of clinical information linked to the document.
3. The method of statement 2 wherein the clinical class is determined from the structured set of clinical information by a clinical class model.
4. The method of statement 3 wherein the clinical class model comprises a sequence of clinical gates, wherein a clinical gate has a criterion and is linked to one of a plurality of clinical classes which is assigned to the document if the structured set of clinical information satisfies the criterion of the clinical gate.
5. The method of statement 3 wherein the clinical class model is a prediction model trained to generate a predicted clinical class from one or more portions of the structured set of clinical information.
6. The method of any of statements 2 to 5 wherein the clinical class is a clinical risk class.
7. The method of any preceding statement further comprising:
   transforming, by the one or more processors, the structured set of clinical information into a graph-based model; and
   extending, by the one or more processors, the graph-based model with a set of one or more nodes of a clinical knowledge graph, wherein the set of one or more nodes are connected to at least one node in the clinical knowledge graph which matches at least one node in the graph-based model.
8. The method of statement 7 further comprising:
   updating, by the one or more processors, the structured set of clinical information based on data linked to the one or more nodes of the clinical knowledge graph.
9. The method of either of statements 7 or 8 wherein the at least one node in the clinical knowledge graph and the at least one node in the graph-based model are linked to a common clinical code.
10. The method of any preceding statement further comprising:
   determining, by the one or more processors, if at least one anomaly is present within the structured set of clinical information based on an anomaly detection model for the clinical context; and
   if at least one anomaly is present within the structured set of clinical information, issuing, by the one or more processors, a warning related to the at least one anomaly.
11. The method of statement 10 wherein the warning is displayed on a user interface viewable by a user.
12. The method of either of statements 10 or 11 wherein the anomaly detection model determines that at least one anomaly is present within the structured set of clinical information if the structured set of clinical information comprises one or more of: a code which is not related to the clinical context; a value outside of an expected range of values; a transcription error; a repeated code; a non-clinical code; incorrect values; a missing expected code; and/ or a missing expected value.
13. The method of any of statements 10 to 12 wherein the anomaly detection model is a rule-based model.
14. The method of any preceding statement further comprising:
   generating, by the one or more processors, a marked-up representation of the document based on the structured set of clinical information, wherein a text portion within the marked-up representation of the document related to a datum of the structured set of clinical information is rendered according to a style linked to a semantic class of the datum.
15. The method of statement 14 further comprising:
   displaying, by the one or more processors, the marked-up representation of the document within a user interface viewable by a user, wherein each rendered text portion is displayed as a selectable element in the user interface.
16. The method of statement 15 wherein the structured set of clinical information is concurrently displayed with the marked-up representation of the document within the user interface, wherein each element of the structured set of clinical information is displayed as a selectable element in the user interface.
17. The method of either of statements 15 or 16 further comprising:
   receiving, by the one or more processors, a user input associated with a first selectable element corresponding to a first rendered text portion related to a first datum of the structured set of clinical information;
   obtaining, by the one or more processors, an updated value for the first datum from a user; and
   updating, by the one or more processors, the first datum in the structured set of clinical information to the updated value.
18. The method of any preceding statement further comprising:
   identifying, by the one or more processors, a patient referred to within the document;
   obtaining, by the one or more processors, an electronic health record linked to the patient; and
   linking, by the one or more processors, the structured set of clinical information with one or more elements of the electronic health record.
19. The method of any preceding statement wherein the clinical datum assigned to the structured set of clinical information is one of a predefined code or a value.
20. The method of statement 19 wherein the predefined code is derived from decision logic executed as a result of executing the prior sequence of branch nodes connected to the coding node.
21. The method of either of statements 19 or 20 wherein the predefined code is one of: a Systematized Nomenclature of Medicine Clinical Terms (SNOMED CT) code; an International Classification of Disease (ICD)-9 code; an ICD-10 code; an ICD-11 code; a Healthcare Common Procedure Coding System (HCPCS) code; a Current Procedure Terminology (CPT) code; a medical prescription normalised Medical prescription (RxNorm) code; a Logical Observation Identifiers Names and Codes (LOINC) code; a Medical Subject Headings (MeSH) code; or a Unified Medical Language System (ULMS) code.
22. The method of any of statements 19 to 21 wherein the value is extracted from the text-based representation of the document.
23. The method any preceding statement wherein the text-based representation of the document is obtained by an optical character recognition process or a multi-modal generative model.
24. The method of any preceding statement wherein the semantic analysis comprises providing a prompt to a large language model (LLM) to determine the evaluation of the query, wherein the prompt comprises a predefined command portion and a context portion comprising at least a part of the text-based representation of the document.
25. The method of any of statements 1 to 23 wherein the semantic analysis comprises processing at least a portion of the text-based representation of the document using a custom natural language processing function.
26. The method of any preceding statement wherein the clinical context of the document is linked to a clinical domain of the document and a type of the document.
27. A computer-readable medium comprising instructions which, when executed by one or more processors, cause the one or more processors to carry out the steps of any of statements 1 to 26
28. A system comprising:
   a memory storing a text-based representation of a document having a clinical context;
   a document classification module comprising a classifier trained to output a predicted clinical identifier from text provided as input;
   a coding graph module configured to identify a respective executable coding graph from a plurality of executable coding graphs based on a respective clinical context, wherein
   each of the plurality of executable coding graphs is indicative of a procedure for coding a clinical document according to a corresponding clinical context and comprises a network of branch nodes interconnected with a plurality of coding nodes thereby forming a directed acyclic graph, wherein:
      a branch node of the network of branch nodes is operable to determine which node connected to the branch node is to be executed next according to an evaluation of a query related to the corresponding clinical context and linked to the branch node, wherein the query is evaluated based on a semantic analysis of the clinical document; and
      a coding node of the plurality of coding nodes is operable to assign a clinical datum to a structured set of clinical information linked to the clinical document, wherein the clinical datum is determined from the semantic analysis performed by executing a prior sequence of branch nodes connected to the coding node; and
   an orchestration module configured to:
      provide one or more portions of the text-based representation of the document to the document classification module to determine an identifier for the document;
      provide the identifier to the coding graph module to identify an executable coding graph for the clinical context; and
      execute the executable coding graph on the text based representation of the document thereby generating the structured set of clinical information linked to the document.

## Claims

1. A method for context-based clinical knowledge extraction, the method comprising:
obtaining, by one or more processors, a text-based representation of a document having a clinical context;
determining, by the one or more processors, an identifier which uniquely identifies the clinical context of the document by providing one or more portions of the text-based representation of the document to a classifier trained to generate a predicted identifier from text provided as input;
identifying, by the one or more processors, an executable coding graph from a plurality of executable coding graphs based on the identifier of the clinical context, the executable coding graph indicative of a procedure for coding the document according to the clinical context and comprising a network of branch nodes interconnected with a plurality of coding nodes thereby forming a directed acyclic graph, wherein:
a branch node of the network of branch nodes is operable to determine which node connected to the branch node is to be executed next according to an evaluation of a query related to the clinical context and linked to the branch node, wherein the query is evaluated based on a semantic analysis of the text-based representation of the document; and
a coding node of the plurality of coding nodes is operable to assign a clinical datum to a structured set of clinical information linked to the document, wherein the clinical datum is determined from the semantic analysis performed by executing a prior sequence of branch nodes connected to the coding node; and
executing, by the one or more processors, the executable coding graph on the text-based representation of the document thereby generating the structured set of clinical information linked to the document.

2. The method of claim 1 further comprising:
transmitting, by the one or more processors and along a communication channel, the document to a recipient entity associated with a clinical class identified from the structured set of clinical information linked to the document.

3. The method of claim 2 wherein the clinical class is determined from the structured set of clinical information by a clinical class model.

4. The method of claim 3 wherein the clinical class model comprises a sequence of clinical gates, wherein a clinical gate has a criterion and is linked to one of a plurality of clinical classes which is assigned to the document if the structured set of clinical information satisfies the criterion of the clinical gate.

5. The method of any preceding claim further comprising:
transforming, by the one or more processors, the structured set of clinical information into a graph-based model; and
extending, by the one or more processors, the graph-based model with a set of one or more nodes of a clinical knowledge graph, wherein the set of one or more nodes are connected to at least one node in the clinical knowledge graph which matches at least one node in the graph-based model.

6. The method of claim 5 further comprising:
updating, by the one or more processors, the structured set of clinical information based on data linked to the one or more nodes of the clinical knowledge graph.

7. The method of any preceding claim further comprising:
determining, by the one or more processors, if at least one anomaly is present within the structured set of clinical information based on an anomaly detection model for the clinical context; and
if at least one anomaly is present within the structured set of clinical information, issuing, by the one or more processors, a warning related to the at least one anomaly.

8. The method of any preceding claim further comprising:
generating, by the one or more processors, a marked-up representation of the document based on the structured set of clinical information, wherein a text portion within the marked-up representation of the document related to a datum of the structured set of clinical information is rendered according to a style linked to a semantic class of the datum.

9. The method of claim 8 further comprising:
displaying, by the one or more processors, the marked-up representation of the document within a user interface viewable by a user, wherein each rendered text portion is displayed as a selectable element in the user interface.

10. The method of claim 9 further comprising:
receiving, by the one or more processors, a user input associated with a first selectable element corresponding to a first rendered text portion related to a first datum of the structured set of clinical information;
obtaining, by the one or more processors, an updated value for the first datum from a user; and
updating, by the one or more processors, the first datum in the structured set of clinical information to the updated value.

11. The method of any preceding claim further comprising:
identifying, by the one or more processors, a patient referred to within the document;
obtaining, by the one or more processors, an electronic health record linked to the patient; and
linking, by the one or more processors, the structured set of clinical information with one or more elements of the electronic health record.

12. The method of any preceding claim wherein the semantic analysis comprises providing a prompt to a large language model (LLM) to determine the evaluation of the query, wherein the prompt comprises a predefined command portion and a context portion comprising at least a part of the text-based representation of the document.

13. The method of any preceding claim wherein the clinical context of the document is linked to a clinical domain of the document and a type of the document.

14. A computer-readable medium comprising instructions which, when executed by one or more processors, cause the one or more processors to carry out the steps of any of claims 1 to 13

15. A system comprising:
a memory storing a text-based representation of a document having a clinical context;
a document classification module comprising a classifier trained to output a predicted clinical identifier from text provided as input;
a coding graph module configured to identify a respective executable coding graph from a plurality of executable coding graphs based on a respective clinical context, wherein each of the plurality of executable coding graphs is indicative of a procedure for coding a clinical document according to a corresponding clinical context and comprises a network of branch nodes interconnected with a plurality of coding nodes thereby forming a directed acyclic graph, wherein:
a branch node of the network of branch nodes is operable to determine which node connected to the branch node is to be executed next according to an evaluation of a query related to the corresponding clinical context and linked to the branch node, wherein the query is evaluated based on a semantic analysis of the clinical document; and
a coding node of the plurality of coding nodes is operable to assign a clinical datum to a structured set of clinical information linked to the clinical document, wherein the clinical datum is determined from the semantic analysis performed by executing a prior sequence of branch nodes connected to the coding node; and
an orchestration module configured to:
provide one or more portions of the text-based representation of the document to the document classification module to determine an identifier for the document;
provide the identifier to the coding graph module to identify an executable coding graph for the clinical context; and
execute the executable coding graph on the text based representation of the document thereby generating the structured set of clinical information linked to the document.
